(19) Europäisches Patentamt
European Patent Office
Office européen des brevets

(11) EP 0 726 899 B1

(12) **EUROPÄISCHE PATENTSCHRIFT**

(45) Veröffentlichungstag und Bekanntmachung des Hinweises auf die Patenterteilung:
**19.01.2000 Patentblatt 2000/03**

(21) Anmeldenummer: **94931018.9**

(22) Anmeldetag: **31.10.1994**

(51) Int. Cl.⁷: **C07D 403/12**, C07D 487/04, C07D 471/04, C07D 401/12, C07D 241/34, A61K 31/495 // (C07D471/04, 231:00, 221:00), (C07D487/04, 241:00, 235:00), (C07D487/04, 239:00, 209:00)

(86) Internationale Anmeldenummer:
**PCT/EP94/03580**

(87) Internationale Veröffentlichungsnummer:
**WO 95/12592 (11.05.1995 Gazette 1995/20)**

(54) **NEUE PYRAZINCARBOXAMIDDERIVATE, IHRE HERSTELLUNG UND IHRE VERWENDUNG IN ARZNEIMITTELN**

NOVEL PYRAZINE CARBOXAMIDE DERIVATIVES, THEIR PRODUCTION AND THEIR USE IN MEDICAMENTS

NOUVEAUX DERIVES DE PYRAZINECARBOXAMIDE, LEUR PREPARATION ET LEUR UTILISATION DANS DES MEDICAMENTS

(84) Benannte Vertragsstaaten:
**AT BE CH DE DK ES FR GB GR IE IT LI LU MC NL PT SE**

(30) Priorität: **04.11.1993 DE 4337609**

(43) Veröffentlichungstag der Anmeldung:
**21.08.1996 Patentblatt 1996/34**

(73) Patentinhaber:
- **Boehringer Ingelheim Pharma KG**
  **55216 Ingelheim am Rhein (DE)**
  Benannte Vertragsstaaten:
  **BE CH DE DK ES FR GR IT LI LU MC NL PT SE AT**
- **BOEHRINGER INGELHEIM INTERNATIONAL GmbH**
  **55218 Ingelheim am Rhein (DE)**
  Benannte Vertragsstaaten:
  **GB IE**

(72) Erfinder:
- **ROOS, Otto**
  **D-55270 Schwabenheim (DE)**
- **SPECK, Georg**
  **D-55218 Ingelheim (DE)**
- **LÖSEL, Walter**
  **D-55435 Gau-Algesheim (DE)**
- **ARNDTS, Dietrich**
  **D-55437 Appenheim (DE)**
- **BECHTEL, Wolf-Dietrich**
  **D-55437 Appenheim (DE)**

(74) Vertreter: **Laudien, Dieter, Dr.**
**Boehringer Ingelheim GmbH,**
**Binger Strasse 173**
**55216 Ingelheim (DE)**

(56) Entgegenhaltungen:
**WO-A-93/04048** **US-A- 3 313 813**

- **MOLECULAR PHARMACOLOGY, Bd.30, Nr.2, 1986 Seiten 112 - 120 L.SIMCHOWITZ ET AL 'Inhibition of Chemotactic Factor-activated Na+/H+ exchange in Human Neutrophils by analogues of Amiloride'**
- **BIOCHEMISTRY, Bd.24, Nr.6, 1985 Seiten 1394 - 1403 G.KACZOROWSKI 'Inhibition of Na+/Ca2+ exchange in pituitary plasma membrane vesicles by analogues of Amiloride'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 726 899 B1

## Beschreibung

Die Erfindung betrifft neue Pyrazincarboxamidderivate, ihre Herstellung nach konventionellen Methoden und ihre Verwendung in bzw. bei der Herstellung von Arzneimitteln.

Die neuen Verbindungen entsprechen der Formel

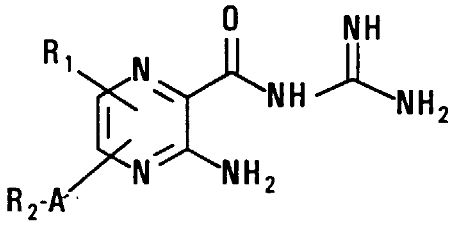

(I)

und können als Basen oder als Salze mit Säuren vorliegen.

In der Formel I steht

A für eine der zweibindigen stets über ein Stickstoffatom an das Pyrazincarboxamidsystem gebundenen Gruppen

$-C_mH_{2m}-NR_4-$ $-C_mH_{2m}-NR_4-C_pH_{2p}-NR_4'-$ $-NR_4-C_mH_{2m}-NR_4'$

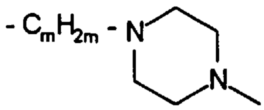

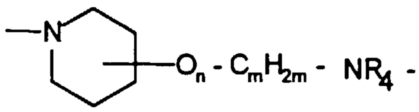

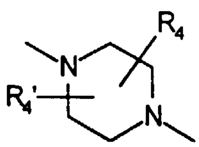

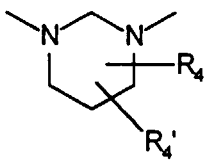

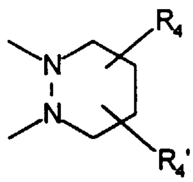

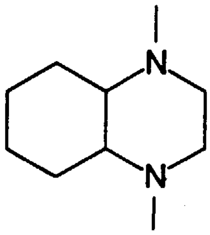 , 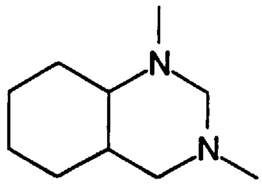 , 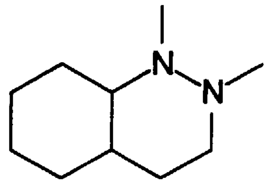 ,

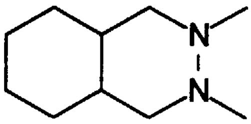 , 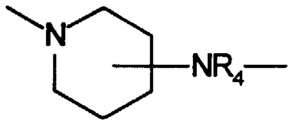 , 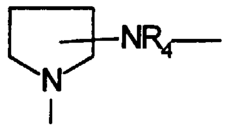 ,

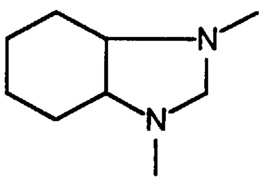 , 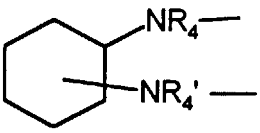 , 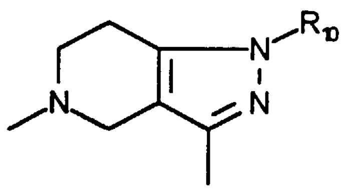 ,

R1 für Wasserstoff, Fluor, Chlor, $C_1$-$C_4$-Alkyl, $C_1$-$C_4$-Alkkoxy, Trifluormethyl, Phenyl, Benzyl, Phenyl-niederalkyl, Phenyl-niederalkenyl, Phenyl-niederalkinyl, Phenoxy, wobei die jeweiligen Phenylgruppen bis zu drei Substituenten aus der Gruppe $C_1$-$C_4$-Alkyl, $C_1$-$C_4$-Alkoxy, $CF_3$, F, CL, CN aufweisen können, oder $C_3$-$C_7$-Cycloalkyl;

R2 für einen Rest der Formel

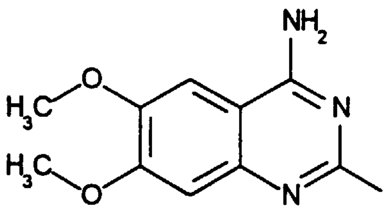 (II)

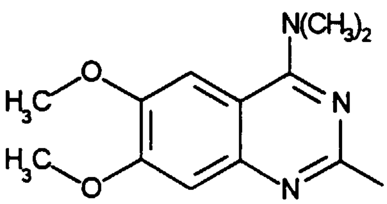 (III)

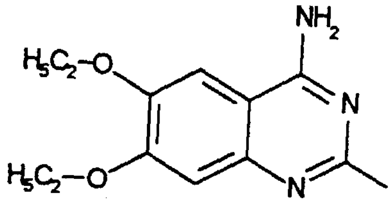

(IV)

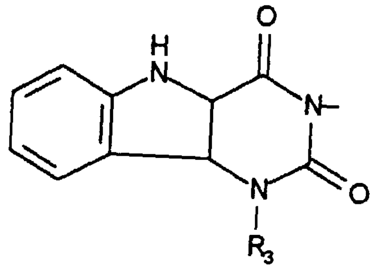

(V)

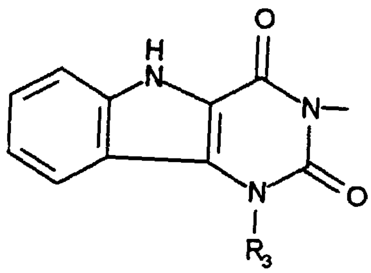

(VI)

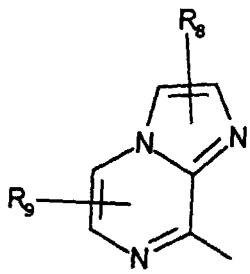

(VII)

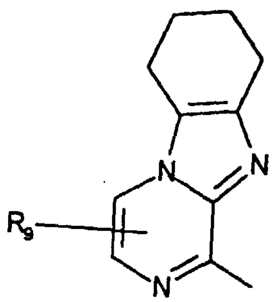

(VIII)

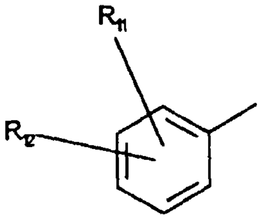

(IX)

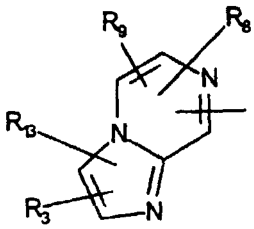

(X)

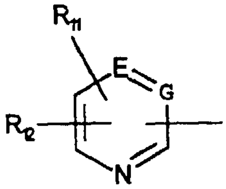

(XI)

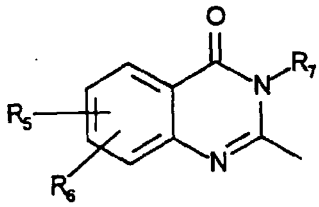

(XII)

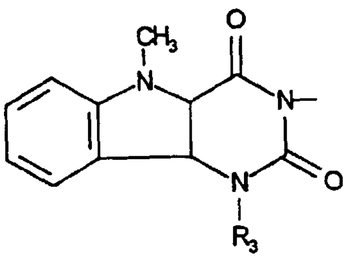

(XIII)

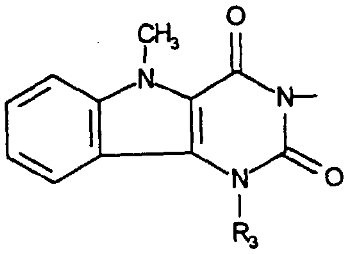

(XIV)

worin

$R_3$, $R_4$ und $R_4$', die gleich oder verschieden sein können, Wasserstoff, $C_1$-$C_4$-Alkyl, $R_4$ und $R_4$' auch Phenyl, Benzyl

und $C_3$-$C_7$-Cycloalkyl,

$R_5$ und $R_6$, die gleich oder verschieden sein können, Wasserstoff, Methyl, Methoxy, Hydroxy oder Halogen,

$R_7$ Wasserstoff, $C_1$-$C_4$-Alkyl, Benzyl oder Benzyloxy,

$R_8$ und $R_9$, die gleich oder verschieden sein können, Wasserstoff, $C_1$-$C_4$-Alkyl, Phenyl oder Halogen,

$R_{10}$ Wasserstoff oder $C_1$-$C_6$-Alkyl, das auch durch Phenyl oder methyl-, methoxy- oder halogensubstituiertes Phenyl substituiert sein kann,

$R_{11}$ und $R_{12}$, die gleich oder verschieden sein können, Wasserstoff, Methyl, Methoxy, Phenyl, Benzyl, Nitro, Cyano, Halogen, Trifluormethyl, Amino, $NR_{10}R_{13}$, 1-Pyrrolidinyl, 1-Pyrazolinyl, 1-Imidazolidinyl, 1-Piperidinyl, 1-Piperazinyl oder Carbamoyl, $R_{11}$ auch einen ankondensierten Benzolring, der bis zu drei Substituenten aus der Gruppe Methyl, Methoxy, Halogen, $CF_3$ und CN aufweisen kann,

$R_{13}$ Wasserstoff, $C_1$-$C_4$-Alkyl, das auch durch Phenyl, Phenoxy oder Benzyloxy substituiert sein kann, oder Halogen,und

E und G, die gleich oder verschieden sein können, N oder CH,

m 2, 3, 4, 5 oder 6,

n 0 oder 1 und

p 2, 3 oder 4 bedeuten
mit der Maßgabe, daß im Falle

$R_1$ Chlor bedeutet und sich in para-Stellung zu $NH_2$-Substituenten am Phenylsystem befindet und

A die Bedeutung von —$C_mH_{2m}$-$NR_4$- oder HN-$CH_2$-$CH_2$-NH

$R_4$ Wasserstoff oder $C_1$-$C_4$-Alkyl bedeutet

$R_2$ nicht einen Rest der Formel (II), (III), (IV), (V), (VI), (VII), (VIII), (X), (XI), (XII), (XIII) oder (XIV) bedeuten darf;
sowie mit der Maßgabe, daß im Falle

$R_1$ Chlor bedeutet und sich in para-Stellung zum $NH_2$-Substituenten am Phenylsystem befindet um

A für einen Piperazinring

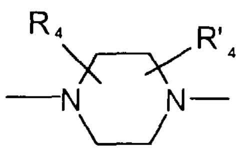

steht, in dem $R_4$ und $R_4$' Wasserstoff bedeuten

$R_2$ nicht einen Rest der Formel (II), (III), (IV), (V), (VI), (VII), (VIII), (X), (XI), (XII), (XIII) oder (XIV) bedeuten darf;
sowie mit der Maßgabe, daß im Falle

$R^1$ Chlor bedeutet und sich in para-Stellung zum $NH_2$-Substituenten am Phenyl-System befindet

A für eine -$CH_2$-$CH_2$-NH-Brücke steht

$R_2$ nicht die Bedeutung von Phenyl haben darf.

Die in den obigen Definitionen genannten Alkyl-, Alkenyl-, Alkinyl- oder Alkylengruppen können geradkettig oder verzweigt sein. Als "niedere" Gruppen sind solche mit 1 bis 4, insbesondere 1 bis 3, vor allem 1 bis 2 C-Atomen bezeichnet. Von den Halogenen sind Fluor, Chlor und Brom, vor allem Fluor und Chlor bevorzugt. Bevorzugte ungesättigte Kohlenwasserstoffreste sind Allyl und Propargyl. Der Index m steht vorzugsweise für 2, 3 oder 4, p für 2 oder 3.
Soweit die neuen Verbindungen in verschiedenen stereoisomeren bzw. cis-/trans-isomeren Formen existieren können, sind mit den obigen Formeln die reinen Formen wie auch ihre Mischungen gemeint.
Die Gruppen $R_2$-A- haben typischerweise Strukturen wie die folgenden:

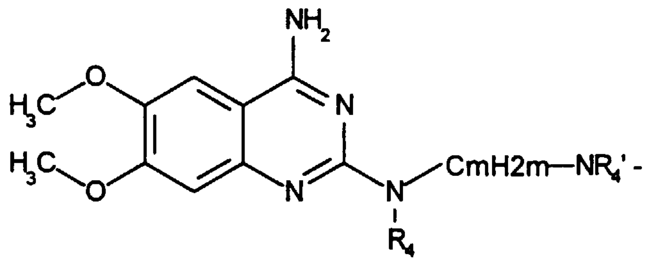

(II')

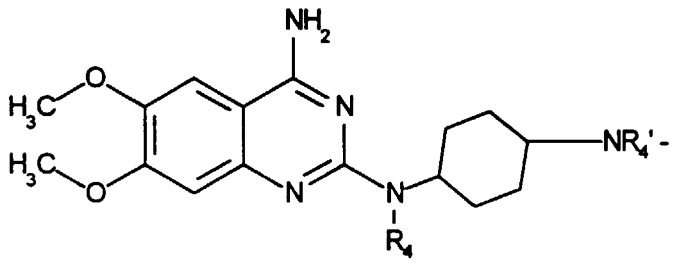

(III')

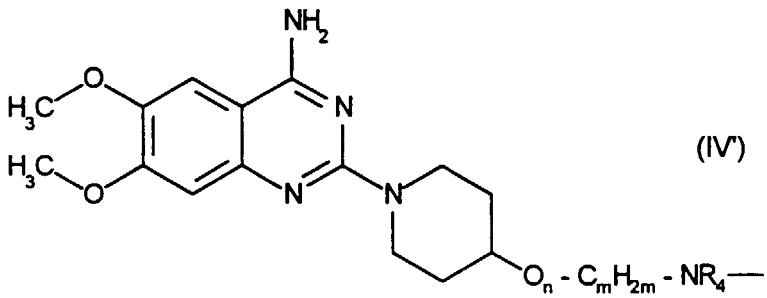

(IV')

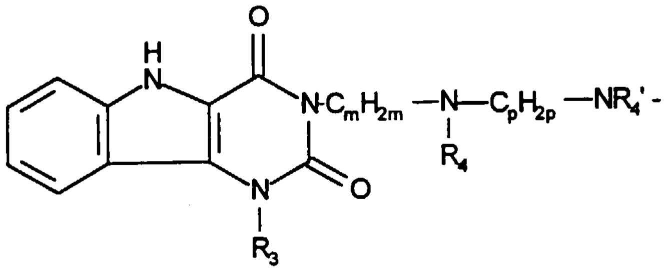

(V')

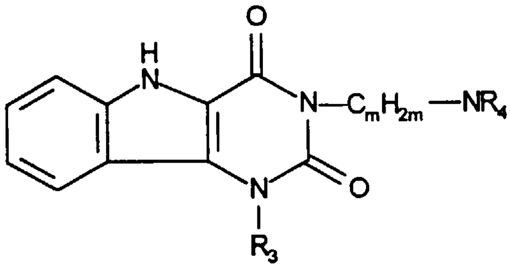

(VI')

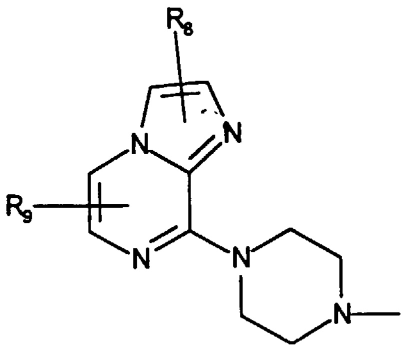

(VII')

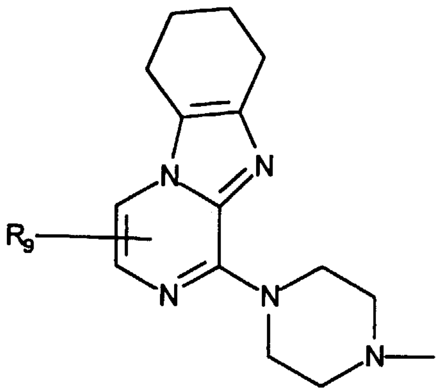

(VIII')

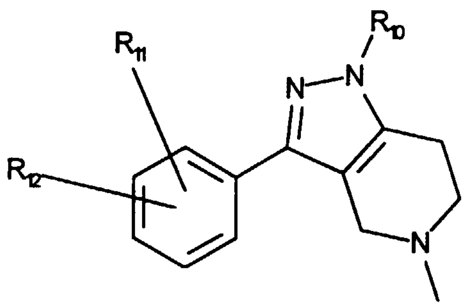

(IX')

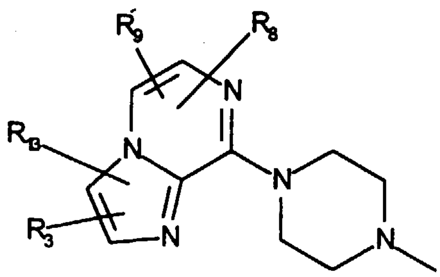

(X')

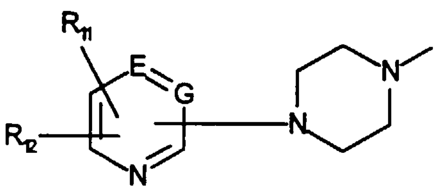

(XI')

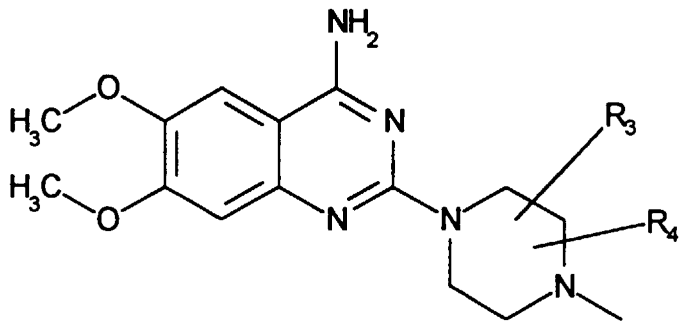

(XII')

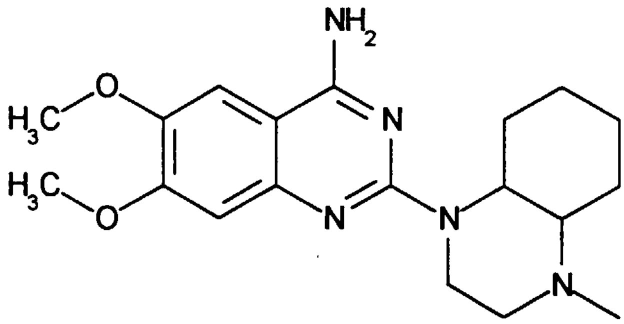

(XIII')

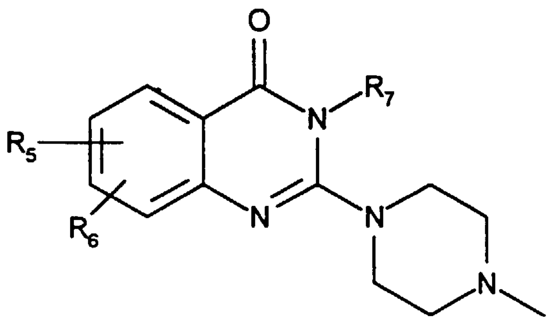

(XIV')

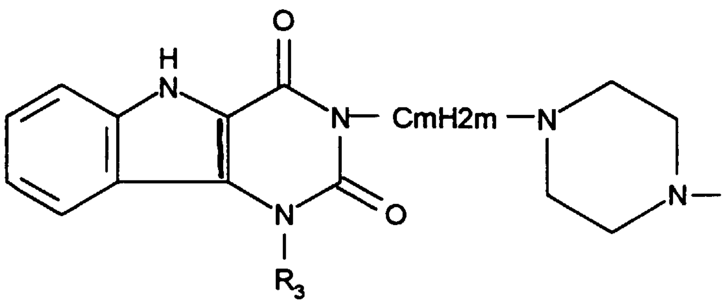

(XV')

wenn A an ein Stickstoffatom des Restes $R_2$ gebunden ist, liegt im allgemeinen ein solcher Rest A vor, in dem die Bindung an $R_2$ von einem C-Atom ausgeht wie in II', III' und IV'.

Verbindungen dieses Typs sind bereits aus dem Stand der Technik bekannt. So werden in der Internationalen Patenanmeldung WO-A-93/04048 sowie in dem US-Patent Nr. 3 313 813 Verbindungen mit identischem Grundkörper beschrieben. Daneben ist aus dem Stand der Technik bekannt [L. Simchowitz und E.J. Gragoe Jr., Molecular Pharmacology, 30 (1986) 113 und G.J. Kaczorowski et al., Biochemistry 24 (1984) 1394], daß derartige Pyrazinabkömmlinge eine diuretische Aktivität besitzen und eine Hemmung des $Na^+/H^+$-Austausches bewirken.

Die Aufgabe, weiche der vorliegenden Erfindung zugrunde liegt, besteht darin, Pyrazinderivate zur Verfügung zu stellen, die sich - gegenüber denjenigen aus dem Stand der Technik bekannten Wirkstoffen - durch eine verbesserte Wirksamkeit auszeichnen. Gelöst wird diese Aufgabe durch die erfindungsgemäß vorgeschlagenen Pyrazincarboxamid-Derivate.

Die neuen Verbindungen werden nach üblichen Methoden erhalten, insbesondere nach den folgenden Verfahren.

1. Umsetzung einer Verbindung der Formel

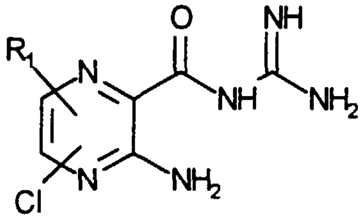

(XVI)

mit einem Amin der Formel

$$R_2 - A - H \qquad (XVII)$$

wobei $R_1$ und $R_2$ die oben angegebene Bedeutung haben.

Die Umsetzung erfolgt in einem polaren, möglichst wasserfreien Lösungsmittel oder Lösungsmittelgemisch, insbesondere Dimethylsulfoxid, Dimethylformamid, vorzugsweise in Gegenwart einer Base, etwa Triethylamin, N-Methylpiperidin, Pyridin, in der Wärme.

2. Zur Herstellung solcher Verbindungen, in denen $R_2$ einen Rest der Formel II, III oder IV darstellt, kann die Verknüpfung auch über ein anderes Stickstoffatom des Restes $R_2$ erfolgen. Beispielsweise werden die Verbindungen mit $R_2$ gleich II auch erhalten, indem man ein Amin der Formel

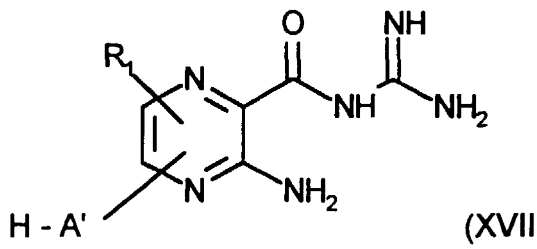

(XVIII)

(wobei A' eine solche Gruppe A ist, die über ein N-Atom an R2 gebunden ist, wie in II', III' und IV')
(worin $R_1$ die obige Bedeutung hat) mit dem Chinazolinderivat der Formel

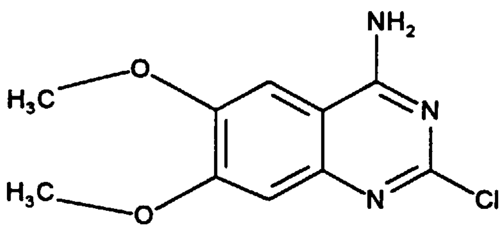

(XIX)

wie unter 1. beschrieben, umsetzt.

3. Umsetzung einer Verbindung der Formel

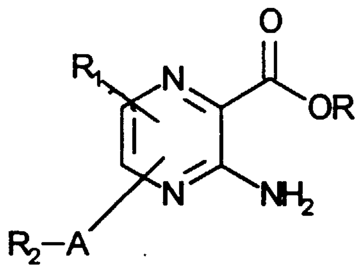

(XX)

(R niederer Alkylrest, Benzyl)

mit Guanidin. Die Umsetzung ist nicht auf die Ester mit R in den genannten Bedeutungen beschränkt, doch wird der Fachmann zweckmäßig einen gut herstellbaren Ester, etwa den Methyl- oder Ethylester verwenden, bzw. einen Ester, bei dessen Umsetzung ein unproblematischer Alkohol entsteht.

Bevorzugt verwendet man den Alkohol, der auch in der Estergruppe enthalten ist, indem man z.B. einen Methylester der Formel XX in Methanol bei Siedetemperatur umsetzt. Dieses Verfahren eignet sich am besten zur Herstellung der erfindungsgemäßen Verbindungen.

Soweit die Verbindungen in stereoisomeren Formen vorliegen können, werden entsprechende Ausgangsprodukte eingesetzt oder es werden ggf. bei der Herstellung gebildete Mischungen in die Komponenten aufgetrennt.

Die Ausgangsstoffe können, soweit sie nicht schon bekannt sind, ebenfalls nach konventionellen Verfahren erhalten werden. Werden beispielsweise anstelle der N-Amidino-carboxamide der Formel XVI bzw. der Formel XVII die entsprechenden Ester eingesetzt, so gelangt man zu den Ausgangsstoffen der Formel XX.

Die Verbindungen der Formel I sind als Wirkstoffe in Arzneimitteln verwendbar oder können als Zwischenprodukte zur Herstellung solcher Wirkstoffe Verwendung finden. Unter anderem hemmen die neuen Verbindungen den $Na^+/H^+$- und den $Na^+/Li^+$-Austausch. Die erfindungsgemäßen Wirkstoffe können als Antihypertensiva, Mucolytika, Diuretika und Cancerostatika benutzt werden; sie sind ferner anwendbar bei Krankheiten, die im Zusammenhang mit Ischämien stehen (Beispiele: cardiale, cerebrale, gastrointestinale, pulmonale, renale Ischämie, Ischämie der Leber, Ischämie der Skelettmulskulatur). Entsprechende Krankheiten sind beispielsweise coronare Herzkrankheit, Angina pectoris, Embolie im Lungenkreislauf, akutes oder chronisches Nierenversagen, chronische Niereninsuffizienz, Hirninfarkt (z. B. nach der Wiederdurchblutung von Hirnarealen nach Auflösung von Gefäßverschlüssen, auch in Kombination mit t-PA, Streptokinases, Urokinase usw.), akute und chronische Durchblutungsstörungen des Hirns. Bei der Reperfusion des ischämischen Herzens (z.B. nach einem Angina-pectoris-Anfall oder einem Herzinfarkt) können irrevesible Schädigungen an Cardiomyocyten in der betroffenen Region auftreten. Die erfindungsgemäßen Verbindungen können u.a. in einem solchen Fall zur Cardioprotektion benutzt werden. Die neuen Verbindungen zeichnen sich durch geringe Nebenwirkungen aus, wobei das weitgehende Fehlen einer $\alpha_1$— und/oder $\alpha_2$—Wirkung erwähnenswert ist.

In das Anwendungsgebiet Ischämie ist auch die Verhinderung von Schäden an Transplantaten einzubeziehen (z. B. als Schutz des Transplantats vor, während und nach der Implantation), die im Zusammenhang mit Transplantationen auftreten können.

Zur Anwendung der Wirkstoffe eignen sich übliche Formulierungen, etwa Tabletten Dragées, Kapseln, Granulate, Injektionslösungen, ggf. auch nasal applizierbare Zubereitungen, wobei die Menge der Wirksubstanz in einer Einzelgabe im allgemeinen 1 bis 200 mg, vorzugsweise 20-100 mg beträgt.

Die Herstellung dieser Arzneimittelformen erfolgt in an sich bekannter Weise.

<u>Beispiele</u>

1. Tabletten (Zusammensetzung)

| | |
|---|---|
| Verbindung nach Beispiel | 40,0 mg |
| Maisstärke | 144,0 mg |

(fortgesetzt)

| | |
|---|---|
| sek. Calciumphosphat | 115,0 mg |
| Magnesiumstearat | 1,0 mg |
| | 300,0 mg |

2. Gelatinekapseln

Der Inhalt einer Kapsel besteht aus 50,0 mg einer Verbindung gemäß der Erfindung und 150,0 mg Maisstärke.

Die nachstehenden Synthesebeispiele sollen die Erfindung näher erläutern.

In den darauffolgenden Tabellen sind Verbindungen aufgeführt, die sich von der nachstehenden Formel (Ia) ableiten:

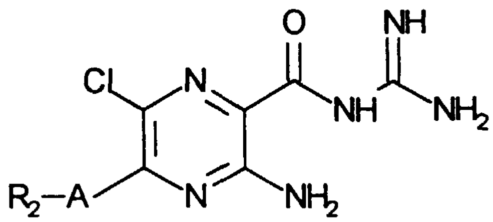

(I a)

Beispiel 1(Methode 1)

N-[2-(6,7-dimethoxy-4-chinazolinonyl)-N'-[5-[2-(N-amidino-carbamoyl)-3-amino-6-chlor]pyrazinyl]-N,N'-piperazin-hydrochlorid

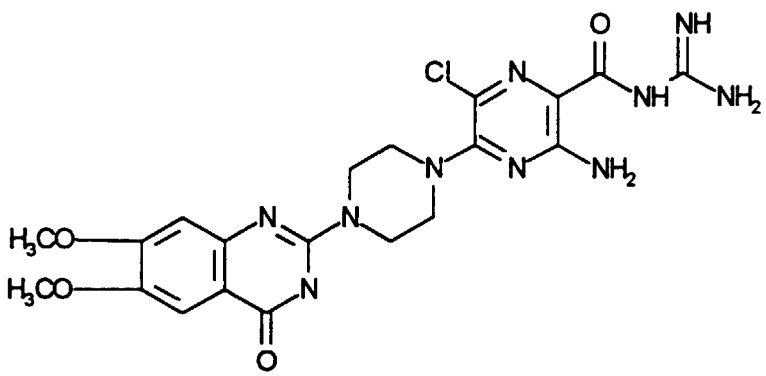

285,5 mg (1 m Mol) 3-Amino-5,6-dichlorpiperazinoyl-2-guanidin-Hydrochlorid werden mit 326,8 mg 3-piperazinyl-(6,7-dimethoxy-chinazolinen-4-Hydrochlorid in 10 ml Dimethylformamid unter Zusatz von 100 mg Triethylamin 2 Std. lang auf 90-100°C erhitzt.

Nach Abkühlen auf Zimmertemperatur wird vom kristllinen Reaktionsprodukt abfiltriert und 480 mg Rohprodukt erhalten; dieses wird in 5 ml Methanol suspendiert und zur Salzbildung mit methanol. Salzsäure angesäuert. Nach Zusatz von 2,5 ml Wasser wird durch Erwärmen eine klare Lösung erhalten, aus der beim Erkalten 350 mg der Titelverbindung kristallin vom Fp. 260-263 °C erhalten werden.

Beispiel 2 (Methode 3)

N-[2-(4-Amino-6,7-dimethoxy)chinazolinyl]-N'-[5-[2-(N-amidino-carbamoyl)-3-amino-6-chlor]pyrazinyl]-N,N'-dimethyl-1,2-diaminoethan-hydrochlorid

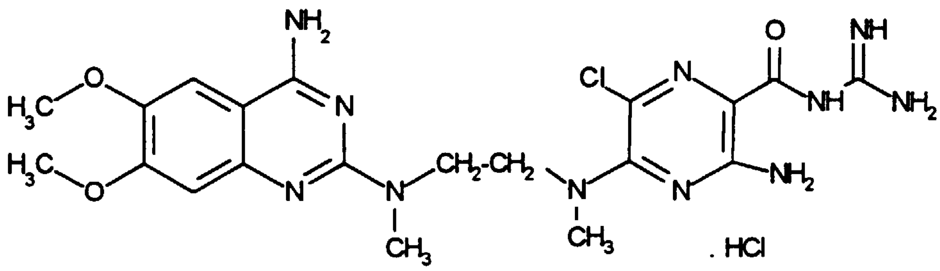

a) 7,2 g (30 m Mol) 1-Amino-3-chlor-6,7-dimethoxychinazolin, 13,2 g (150 m Mol) N,N'-Dimethylethylendiamin werden in 60 ml n-Amylalkohol 1 Std. unter Rühren am Rückfluß erhitzt. Das Lösungsmittel wird im Vakuum abdestilliert, der Rückstand unter Erhitzen in 100 ml Acetonitril gelöst und filtriert. Beim Abkühlen kristallisieren 6,05 g des rohen Zwischenproduktes aus, die in 60 ml kochendem Wasser gelöst und nach Zusatz von 80 ml Aceton umkristallisiert werden konnten. Es werden 3,9 g des reinen N-[(4-Amino-6,7-dimethoxy)-2-chinazolinyl]-N,N' dimethyl-1,2-diaminoethans erhalten.

(b) 5,83 g (20 mmol) N-[(4-Amino-6,7-dimethoxy)-2-chinazolinyl]-N,N'dimethyl-1,2-diaminoethan, 4,44g (20 mmol) 3-Amino-5,6-dichlorpyrazin-2-carbonsäuremethylester, 2,75 ml Triethylamin (20 mmol) werden in 30 ml Dimethylsulfoxid gelöst und 2 Stunden unter Rühren auf 80° C erhitzt. Nach dem Abkühlen wird mit 60 ml Wasser versetzt und das sich abscheidende. Reaktionsprodukt durch Absaugen isoliert. Nach Trocknen wird die Substanz (Ausbeute 9,1 g) ohne weitere Reinigung zum Guanidinderivat umgesetzt.

(c) 9,07 g (95 mmol) Guanidin-hydrochlorid werden mit 95 ml (95 mmol) 1 N methanolischer Natriummethylatlösung 30 Min. bei Raumtemperatur gerührt. Vom ausgefallenen Natriumchlorid wird abgesaugt.
Das Filtrat wird mit einer Lösung von9,1 g (19,1 mmol) 3-Amino-6-chlor-5-[2-[(4-amino-6,7-dimethoxy)-2-chinazolinyl]-1-(N,N'-dimethyl-1,2-diaminoethyl)]-pyrazin-2-carbonsäuremethylester in 50 ml Dimethylformamid versetzt und 3 Stunden auf 100 - 110 °C erhitzt. Nach Abdestillieren des Lösungsmittels wird der verbleibende Rückstand über eine Kieselgel-Säule gereinigt.
Fließmittel: Essigester 70/Isopropanol 30/$NH_4OH$ 10.
Die gereinigte Substanz wird in Ethanol gelöst, mit etherischem Chlorwasserstoff angesäuert und durch Zugabe von Diethylether das Hydrochlorid zur Kristallisation gebracht. Ausbeute: 5,4 g, Fp. 227-30 °C.

Die in den folgenden Tabellen aufgeführten Verbindungen können entsprechend den vorstehenden Beispielen und/oder den Angaben in der Beschreibung erhalten werden.

Tabelle 1

| Verbindungen der Formel I a, worin $R_2$-A- ein Rest der Formel II' ist | | | | |
|---|---|---|---|---|
| Nr. | m | $R_{4'}$ | $R_4$ | Fp. [°C] |
| 1 | 2 | H | H | >220 |
| 2 | 2 | $CH_3$ | H | |
| 3 | 2 | H | $CH_3$ | |

Tabelle 1 (fortgesetzt)

| Verbindungen der Formel I a, worin $R_2$-A- ein Rest der Formel II' ist | | | | |
|---|---|---|---|---|
| Nr. | m | $R_{4'}$ | $R_4$ | Fp. [°C] |
| 4 | 2 | $CH_3$ | $CH_3$ | 227-30 |
| 5 | 2 | $CH_3$ | $C_2H_5$ | |
| 6 | 2 | $C_2H_5$ | $CH_3$ | |
| 7 | 2 | $C_2H_5$ | $C_2H_5$ | |
| 8 | 2 | i-$C_3H_7$ | $CH_3$ | |
| 9 | 2 | $CH_3$ | i-$C_3H_7$ | |
| 10 | 2 | i-$C_3H_7$ | i-$C_3H_7$ | |
| 11 | 3 | H | $CH_3$ | |
| 12 | 3 | $CH_3$ | H | |
| 13 | 3 | $CH_3$ | $CH_3$ | |
| 14 | 3 | $C_2H_5$ | $CH_3$ | |
| 15 | 3 | $CH_3$ | $C_3H_5$ | |
| 16 | 3 | $C_2H_5$ | $C_2H_5$ | |
| 17 | 3 | i-$C_3H_7$ | $CH_3$ | |
| 18 | 3 | $CH_3$ | i-$C_3H_7$ | |
| 19 | 3 | i-$C_3H_7$ | i-$C_3H_7$ | |
| 20 | 6 | $CH_3$ | $CH_3$ | 190-2 |
| 21 | 3 | n-$C_4H_9$ | n-$C_4H_9$ | |

Tabelle 2

| Verbindungen der Formel I a, worin $R_2$-A ein Rest der Formel III' ist | | | | |
|---|---|---|---|---|
| Nr. | $R_{4'}$ | $R_4$ | Form | Fp. [°C] |
| 1 | H | H | cis | |
| 2 | $CH_3$ | H | cis | |
| 3 | H | $CH_3$ | cis | |
| 4 | $CH_3$ | $CH_3$ | cis | |
| 5 | n-$CH_3H_7$ | n-$CH_3H_7$ | cis | |
| 6 | H | H | trans | |
| 7 | $CH_3$ | H | trans | |
| 8 | H | $CH_3$ | trans | |
| 9 | $CH_3$ | $CH_3$ | trans | |
| 10 | n-$CH_3H_7$ | n-$CH_3H_7$ | trans | |
| 11 | i-$C_4H_9$ | H | cis/trans | |
| 12 | H | t-$C_4H_9$ | cis/trans | |

Tabelle 3

| Verbindungen der Formel I a worin $R_2$-A ein Rest der Formel IV' ist | | | | |
|---|---|---|---|---|
| Nr. | m | n | $R_4$ | Fp. [°C] |
| 1 | 2 | 0 | H | |
| 2 | 2 | 0 | $CH_3$ | |
| 3 | 2 | 0 | $C_2H_5$ | |
| 4 | 2 | 0 | i-$C_3H_7$ | |
| 5 | 2 | 0 | $C_6H_5$ | |
| 6 | 2 | 1 | H | |
| 7 | 2 | 1 | $CH_3$ | |
| 8 | 2 | 1 | $C_2H_5$ | |
| 9 | 2 | 1 | i-$C_3H_7$ | |
| 10 | 2 | 1 | $C_6H_5$ | |
| 11 | 3 | 0 | H | |
| 12 | 3 | 0 | $CH_3$ | |
| 13 | 3 | 0 | $C_2H_5$ | |
| 14 | 3 | 0 | i-$C_3H_7$ | |
| 15 | 3 | 0 | $C_6H_5$ | |
| 16 | 3 | 1 | H | |
| 17 | 3 | 1 | $CH_3$ | |
| 18 | 3 | 1 | $C_2H_5$ | |
| 19 | 3 | 1 | i-$C_3H_7$ | |
| 20 | 3 | 1 | $C_6H_5$ | |

Tabelle 4

| Verbindungen der Formel I a worin $R_2$-A ein Rest der Formel V', $R_3$ gleich H und p gleich 2 ist | | | | |
|---|---|---|---|---|
| Nr. | m | $R_4$' | $R_4$ | Fp. [°C] |
| 1 | 2 | H | H | |
| 2 | 2 | $CH_3$ | $CH_3$ | |
| 3 | 2 | $C_2H_5$ | $C_2H_5$ | |
| 4 | 2 | i-$C_3H_7$ | i-$C_3H_7$ | |
| 5 | 2 | $C_6H_5$ | $CH_3$ | |
| 6 | 3 | H | H | |
| 7 | 3 | $CH_3$ | $CH_3$ | |
| 8 | 3 | $C_2H_5$ | $C_2H_5$ | |

Tabelle 4 (fortgesetzt)

| Verbindungen der Formel I a worin $R_2$-A ein Rest der Formel V', $R_3$ gleich H und p gleich 2 ist | | | | |
|---|---|---|---|---|
| Nr. | m | $R_4'$ | $R_4$ | Fp. [°C] |
| 9 | 3 | i-$C_3H_7$ | i-$C_3H_7$ | |
| 10 | 3 | $C_6H_5$ | $CH_3$ | |
| 11 | 2 | n-$C_4H_9$ | $CH_3$ | |
| 12 | 2 | $C_6H_5$ | $C_6H_5$ | |

Tabelle 5

| Verbindungen der Formel I a worin $R_2$-A ein Rest der Formel VI' ist | | | | |
|---|---|---|---|---|
| Nr. | m | $R_4$ | $R_3$ | Fp. [°C] |
| 1 | 2 | H | H | |
| 2 | 2 | $CH_3$ | $CH_3$ | |
| 3 | 2 | $C_2H_5$ | $C_2H_5$ | |
| 4 | 2 | i-$C_3H_7$ | i-$C_3H_7$ | |
| 5 | 2 | $C_6H_5$ | $CH_3$ | |
| 6 | 3 | H | H | |
| 7 | 3 | $CH_3$ | $CH_3$ | |
| 8 | 3 | $C_2H_5$ | $C_2H_5$ | |
| 9 | 3 | i-$C_3H_7$ | i-$C_3H_7$ | |
| 10 | 3 | $C_6H_5$ | $CH_3$ | |
| 11 | 2 | n-$C_4H_9$ | $CH_3$ | |
| 12 | 2 | $C_6H_5$ | $C_6H_5$ | |

**Tabelle 6**

Verbindungen der Formel I a, worin $R_2$-A für einen Rest der Formel VII' steht:

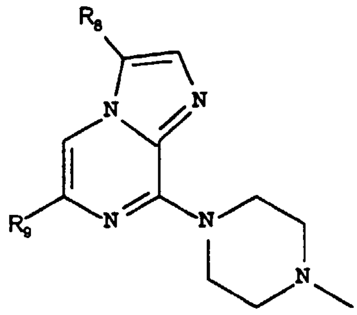

(VII a)

| Nr. | $R_8$ | $R_9$ | Fp. [°C] |
|---|---|---|---|
| 1 | H | H | |
| 2 | $CH_3$ | Cl | |
| 3 | Cl | Cl | |
| 4 | $CH_3$ | $CH_3$ | |

## Tabelle 7

Verbindungen der Formel I a, worin $R_2$-A für einen Rest der Formel VIII' steht:

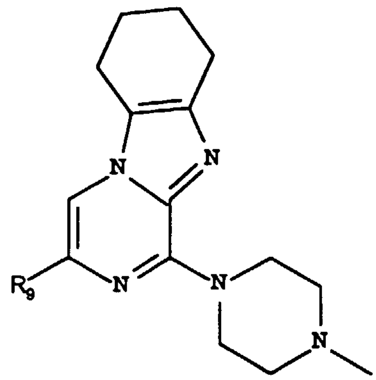

(VIII a)

| Nr. | $R_9$ | Fp. [°C] |
|---|---|---|
| 1 | H | |
| 2 | Cl | |
| 3 | $CH_3$ | |

Tabelle 8

| Verbindungen der Formel I a, worin $R_2$-A ein Rest der Formel IX' ist (die Positionsangaben für $R_{11}$/$R_{12}$ beziehen sich auf den Phenylrest) | | | |
|---|---|---|---|
| Nr. | $R_{10}$ | $R_{11}$ | $R_{12}$ Fp. [°C] |
| 1 | H | H | H |
| 2 | H | 3-Cl | H |
| 3 | H | 2-F | 3-F |
| 4 | H | 4-$NO_2$ | H |
| 5 | H | 4-CN | H |
| 6 | H | 3-$OCH_3$ | 4-$OCH_3$ |
| 7 | H | $CH_3$ | H |
| 8 | H | 4-F | 3-$C_2H_5$ |
| 9 | H | 4-$CH_2$-$C_6H_5$ | H |
| 10 | H | 4-$OCH_3$ | 2-$CH_2$-$C_6N_5$ |
| 11 | $CH_3$ | H | H |

Tabelle 8 (fortgesetzt)

| Verbindungen der Formel I a, worin $R_2$-A ein Rest der Formel IX' ist (die Positionsangaben für $R_{11}$/$R_{12}$ beziehen sich auf den Phenylrest) | | | |
|---|---|---|---|
| Nr. | $R_{10}$ | $R_{11}$ | $R_{12}$ Fp. [°C] |
| 12 | $CH_3$ | 3-Cl | H |
| 13 | $CH_3$ | 2-F | 3-F |
| 14 | $CH_3$ | 4-$NO_2$ | H |
| 15 | $CH_3$ | 4-CN | H |
| 16 | $CH_3$ | 3-$OCH_3$ | 4-$OCH_3$ |
| 17 | $CH_3$ | $CH_3$ | H |
| 18 | $CH_3$ | 4-F | 3-$C_2H_5$ |
| 19 | $CH_3$ | 4-$CH_2$-$C_6H_5$ | H |
| 20 | $CH_3$ | 4-$OCH_3$ | 2-$CH_2$-$C_6N_5$ |
| 21 | $C_2H_5$ | H | H |
| 22 | $C_2H_5$ | 3-Cl | H |
| 23 | $C_2H_5$ | 2-F | 3-F |
| 24 | $C_2H_5$ | 4-$NO_2$ | H |
| 25 | $C_2H_5$ | 4-CN | H |
| 26 | $C_2H_5$ | 3-$OCH_3$ | 4-$OCH_3$ |
| 27 | $C_2H_5$ | $CH_3$ | H |
| 28 | $C_2H_5$ | 4-F | 3-$C_2H_5$ |
| 29 | $C_2H_5$ | 4-$CH_2$-$C_6H_5$ | H |
| 30 | $C_2H_5$ | 4-$OCH_3$ | 2-$CH_2$-$C_6N_5$ |
| 31 | i-$C_3H_7$ | H | H |
| 32 | n-$C_6H_{13}$ | 4-F | H |
| 33 | 4-F-$C_6H_4$ | 4-F | H |
| 34 | $CH_2$-$C_6H_5$ | 4-F | H |
| 35 | $C_2H_5$ | 4-F | H |
| 36 | n-$C_4H_9$ | 2-$CH_3$ | 6-$CH_3$ |

## Tabelle 9

Verbindungen der Formel I a, worin

$R_2$-A einen Rest der Formel X' ist:

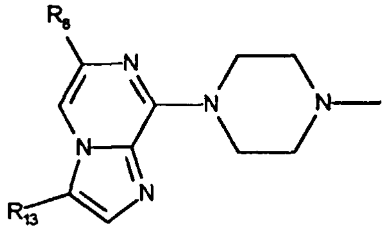

(X a)

($R_3$ und $R_9$ gleich H)

| Nr. | $R_8$ | $R_{13}$ | Fp. [°C] |
|---|---|---|---|
| 1 | H | H | |
| 2 | $C_2H_5$ | H | |
| 3 | H | $CH_3$ | |
| 4 | i-$C_3H_7$ | H | |
| 5 | i-$C_3H_7$ | $CH_3$ | |
| 6 | H | Br | |
| 7 | Br | H | |
| 8 | $CH_3$ | H | |

## Tabelle 9a

Verbindungen der Formel Ia, worin $R_2$-A ein Rest der Formel X' ist:

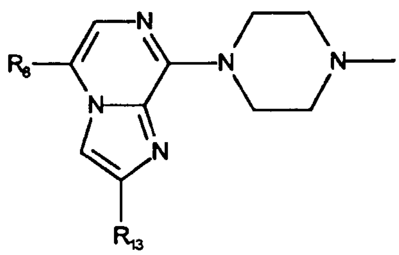

(X b)

($R_3$ und $R_9$ gleich H)

| Nr. | $R_8$ | $R_{13}$ | Fp. [°C] |
|---|---|---|---|
| 1 | H | H | |
| 2 | $C_2H_5$ | H | |
| 3 | H | $CH_3$ | |
| 4 | i-$C_3H_7$ | H | |
| 5 | i-$C_3H_7$ | $CH_3$ | |
| 6 | H | Br | |
| 7 | Br | H | |
| 8 | $CH_3$ | H | |

## Tabelle 10

Verbindungen der Formel Ia, worin $R_2$-A für einen Rest der Formel XI' steht:

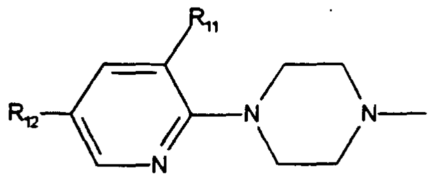

(XI a)

| Nr. | $R_{11}$ | $R_{12}$ | Fp. [°C] |
|---|---|---|---|
| 1 | $NH_2$ | H | 275-8 |
| 2 | $CONH_2$ | H | |
| 3 | Cl | H | |
| 4 | Cl | Cl | |
| 5 | H | H | 288-90 |
| 6 | H | $CH_3$ | |
| 7 | $CH_3$ | H | |
| 8 | CN | H | 239-41 |
| 9 | $CH_3$ | $CH_3$ | |

## Tabelle 11

Verbindungen der Formel I a, worin $R_2$-A für einen Rest der Formel XII' bzw. XIII' :

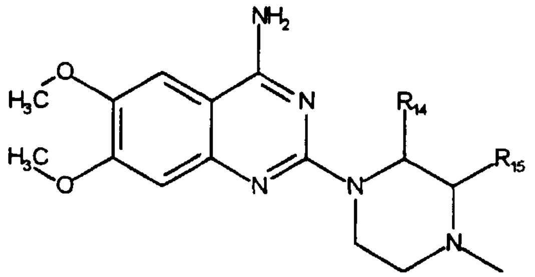

(XII a)

steht, in der $R_{14}$ bzw. $R_{15}$ die Reste $R_3$ bzw. $R_4$ bedeuten, zusätzlich jedoch gemeinsam auch $-CH_2-CH_2-CH_2-CH_2-$

| Nr. | $R_{14}$ | $R_{15}$ | Form | Fp. [°C] |
|---|---|---|---|---|
| 1 | H | $CH_3$ | | |
| 2 | $CH_3$ | H | | |
| 3 | $CH_3$ | $CH_3$ | cis | |
| 4 | $CH_3$ | $CH_3$ | trans | |
| 5 | $C_2H_5$ | $CH_3$ | cis/trans | |
| 6 | $C_2H_5$ | $C_2H_5$ | cis | |
| 7 | $C_2H_5$ | $C_2H_5$ | trans | |
| 8 | H | H | x 2 HCl | 295-310 (Zers.) |
| 9 | H | $i-C_3H_7$ | | |
| 10 | $-CH_2-CH_2-CH_2-CH_2-$ | | cis | |
| 11 | $-CH_2-CH_2-CH_2-CH_2-$ | | trans | |

Tabelle 12

| Verbindungen der Formel I a, worin $R_2$-A für einen Rest der Formel XIV' steht, wobei sich die Positionsangaben für $R_5$ und $R_6$ auf das Chinazolinonringsystem beziehen | | | | |
|---|---|---|---|---|
| Nr. | $R_7$ | $R_5$ | $R_6$ | Fp. [°C] |
| 1 | H | H | H | 260-3 |
| 2 | H | 6-$OCH_3$ | 7-$OCH_3$ | |
| 3 | H | 8-$OCH_3$ | H | |
| 4 | H | 6-OH | 7-OH | |
| 5 | H | 7-OH | H | |
| 6 | H | 8-OH | H | |
| 7 | H | 6-Cl | H | |
| 8 | H | 6-$CH_3$ | 7-$CH_3$ | |
| 9 | $CH_3$ | H | H | |
| 10 | $CH_3$ | 6-$OCH_3$ | 7-$OCH_3$ | |
| 11 | $CH_3$ | 8-$OCH_3$ | H | |
| 12 | $CH_3$ | 6-OH | 7-OH | |
| 13 | $CH_3$ | 7-OH | H | |
| 14 | $CH_3$ | 8-OH | H | |
| 15 | $CH_3$ | 6-Cl | H | |
| 16 | $CH_3$ | 6-$CH_3$ | 7-$CH_3$ | |

Tabelle 13

| Verbindungen der Formel I a, worin $R_2$-A für einen Rest der Formel XV' steht | | | |
|---|---|---|---|
| Nr. | m | $R_3$ | Fp. [°C] |
| 1 | 2 | H | 260-262 |
| 2 | 2 | $CH_3$ | |
| 3 | 2 | H | |
| 4 | 2 | $CH_3$ | |
| 5 | 2 | n-$C_4H_9$ | |
| 6 | 2 | H | |
| 7 | 3 | H | |
| 8 | 3 | $CH_3$ | |
| 9 | 3 | H | |
| 10 | 3 | $CH_3$ | |
| 11 | 3 | n-$C_4H_9$ | |
| 12 | 3 | H | |

## Tabelle 14

Verbindungen der Formel I a mit verschiedenen Resten der Formel $R_2$ und A in der Bedeutung

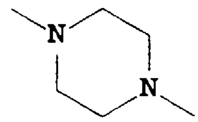

| Nr. | $R_2$ | Fp. [°C] |
|---|---|---|
| 1 | 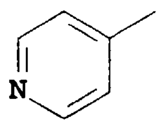 | 290-3 |
| 2 | 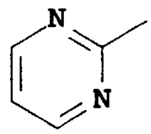 | 268-72 |
| 3 | 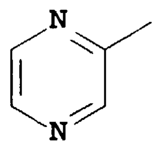 | 235-40 |
| 4 | 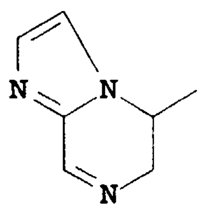 | 205-8 |

## Tabelle 15

Verbindungen der Formel I a mit dem Rest $R_2$ der Formel III und A in den unten angegebenen Bedeutungen

| Nr. | A | Fp. [°C] |
|---|---|---|
| 1 | 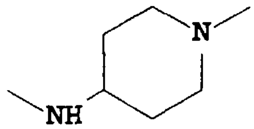 | >280 |
| 2 | 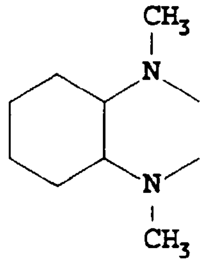 | >280 |

**Patentansprüche**

1. Verbindungen der Formel

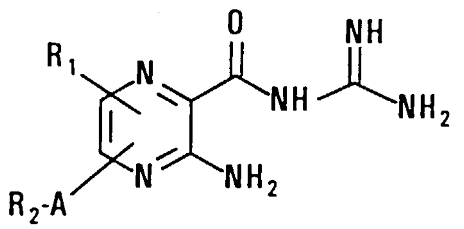

(I)

in der

A für eine der zweibindigen, stets über ein Stickstoffatom an das Pyrazincarboxamidsystem gebundenen Gruppen

$-C_mH_{2m}-NR_4-$ , $-C_mH_{2m}-NR_4-C_pH_{2p}-NR_4'-$ , 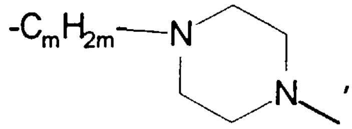 ,

$-NR_4-C_mH_{2m}-NR_4'-$ , 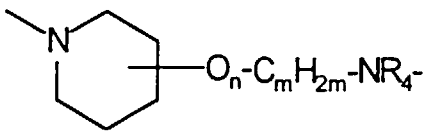 ,

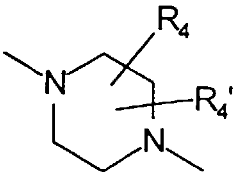 , 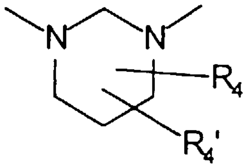 , 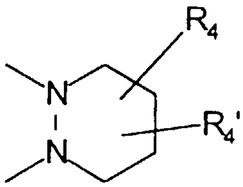 ,

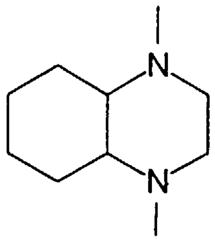 , 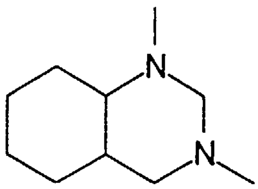 , 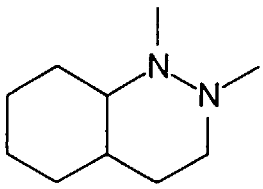 ,

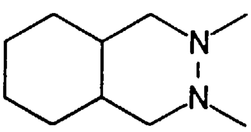 , 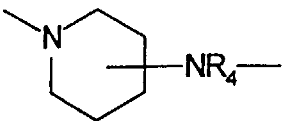 , 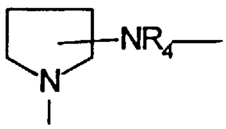 ,

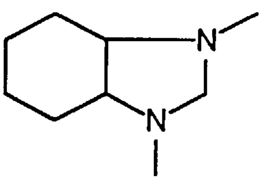 , 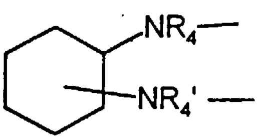 , 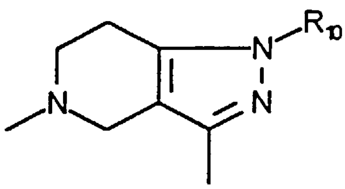 ,

$R_1$ für Wasserstoff, Fluor, Chlor, $C_1$-$C_4$-Alkyl, $C_1$-$C_4$-Alkkoxy, Trifluormethyl, Phenyl, Benzyl, Phenyl-niederalkyl, Phenyl-niederalkenyl, Phenyl-niederalkinyl, Phenoxy, wobei die jeweiligen Phenylgruppen bis zu drei Substituenten aus der Gruppe $C_1$-$C_4$-Alkyl, $C_1$-$C_4$-Alkoxy, $CF_3$, F, CL, CN aufweisen können, oder $C_3$-$C_7$-Cycloalkyl;

$R_2$ für einen Rest der Formel

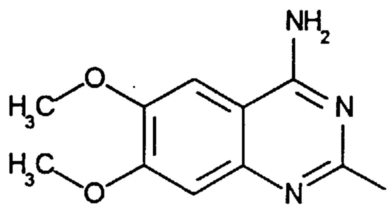

(II)

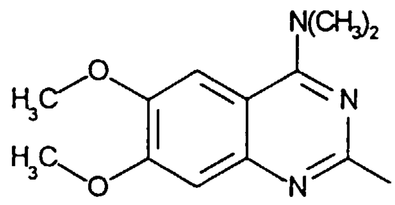

(III)

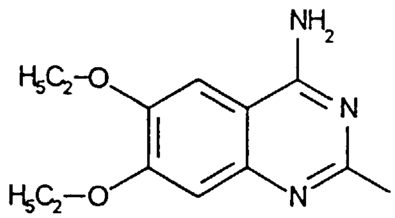

(IV)

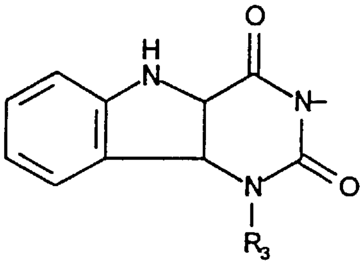

(V)

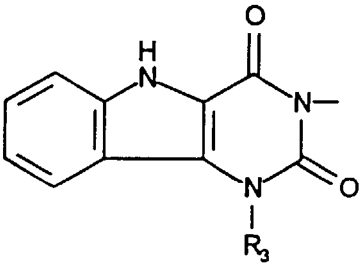

(VI)

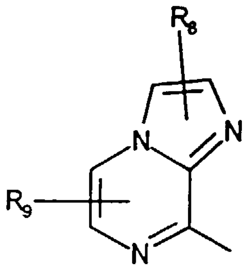

(VII)

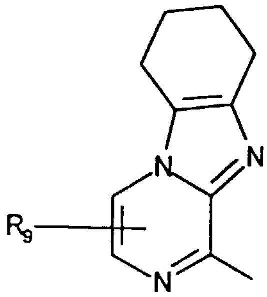

(VIII)

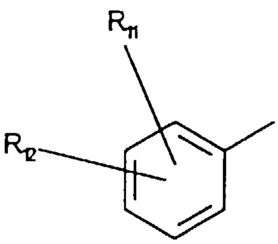

(IX)

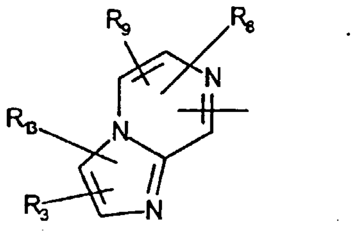

(X)

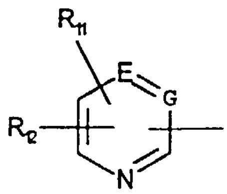

(XI)

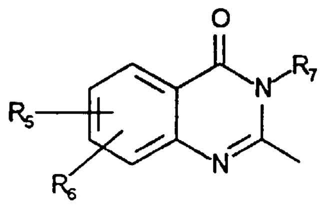

(XII)

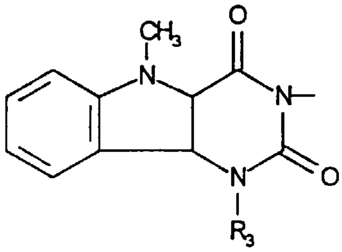

(XIII)

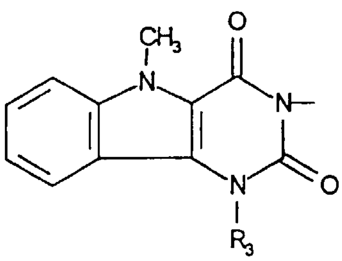

(XIV)

stehen,

worin

R$_3$, R$_4$ und R$_4$', die gleich oder verschieden sein können, Wasserstoff, $C_1$-$C_4$-Alkyl, R$_4$ und R$_4$' auch Phenyl, Benzyl und $C_3$-$C_7$-Cycloalkyl,

| | |
|---|---|
| $R_5$ und $R_6$, | die gleich oder verschieden sein können, Wasserstoff, Methyl, Methoxy, Hydroxy oder Halogen, |
| $R_7$ | Wasserstoff, $C_1$-$C_4$-Alkyl, Benzyl oder Benzyloxy, |
| $R_8$ und $R_9$, | die gleich oder verschieden sein können, Wasserstoff, $C_1$-$C_4$-Alkyl, Phenyl oder Halogen, |
| $R_{10}$ | Wasserstoff oder $C_1$-$C_6$-Alkyl, das auch durch Phenyl oder methyl-, methoxy- oder halogensubstituiertes Phenyl substituiert sein kann, |
| $R_{11}$ und $R_{12}$, | die gleich oder verschieden sein können, Wasserstoff, Methyl, Methoxy, Phenyl, Benzyl, Nitro, Cyano, Halogen, Trifluormethyl, Amino, $NR_{10}R_{13}$, 1-Pyrrolidinyl, 1-Pyrazolinyl, 1-Imidazolidinyl, 1-Piperidinyl, 1-Piperazinyl oder Carbamoyl, $R_{11}$ auch einen ankondensierten Benzolring, der bis zu drei Substituenten aus der Gruppe Methyl, Methoxy, Halogen, $CF_3$ und CN aufweisen kann, |
| $R_{13}$ | Wasserstoff, $C_1$-$C_4$-Alkyl, das auch durch Phenyl, Phenoxy oder Benzyloxy substituiert sein kann, oder Halogen,und |
| E und G, | die gleich oder verschieden sein können, N oder CH, |
| m | 2, 3, 4, 5 oder 6, |
| n | 0 oder 1 und |
| p | 2, 3 oder 4 bedeuten<br>und die Verbindungen der Formel I als Basen oder als Salze und ggf. in Form der einzelnen Stereoisomeren oder cis-/trans-Isomeren bzw. der Mischungen solcher Isomeren vorliegen können<br>mit der Maßgabe, daß im Falle |
| $R_1$ | Chlor bedeutet und sich in para-Stellung zu $NH_2$-Substituenten am Phenylsystem befindet und |
| A | die Bedeutung von $—C_mH_{2m}$-$NR_4$- oder HN-$CH_2$-$CH_2$-NH |
| $R_4$ | Wasserstoff oder $C_1$-$C_4$-Alkyl bedeutet |
| $R_2$ | nicht einen Rest der Formel (II), (III), (IV), (V), (VI), (VII), (VIII), (X), (XI), (XII), (XIII) oder (XIV) bedeuten darf;<br>sowie mit der Maßgabe, daß im Falle |
| $R_1$ | Chlor bedeutet und sich in para-Stellung zum $NH_2$-Substituenten am Phenylsystem befindet um |
| A | für einen Piperazinring<br>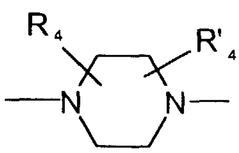<br>steht, in dem $R_4$ und $R_4$' Wasserstoff bedeuten |
| $R_2$ | nicht einen Rest der Formel (II), (III), (IV), (V), (VI), (VII), (VIII), (X), (XI), (XII), (XIII) oder (XIV) bedeuten darf;<br>sowie mit der Maßgabe, daß im Falle |

$R^1$ Chlor bedeutet und sich in para-Stellung zum $NH_2$-Substituenten am Phenyl-System befindet

A für eine $—CH_2$-$CH_2$-NH-Brücke steht

$R_2$ nicht die Bedeutung von Phenyl haben darf.

**2.** Verbindungen der Formel I, in denen $R_2$-A einen Rest der Formel II' bis XV', $R_3$, $R_4$ und $R_4$', die gleich oder verschieden sein können, Wasserstoff oder einen $C_1$-$C_3$-Alkylrest bedeuten

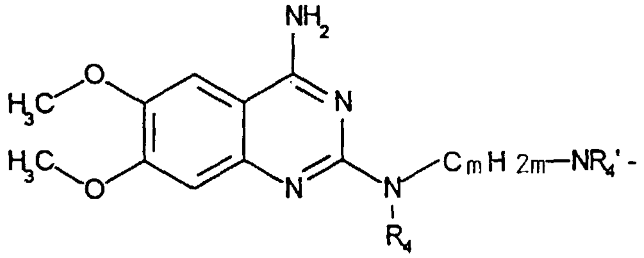

(II')

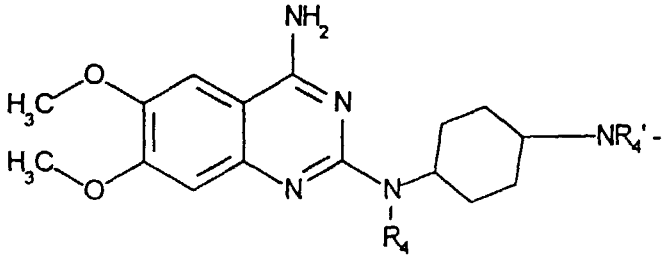

(III')

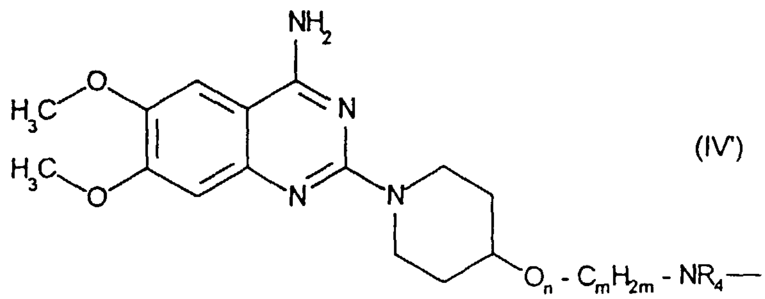

(IV')

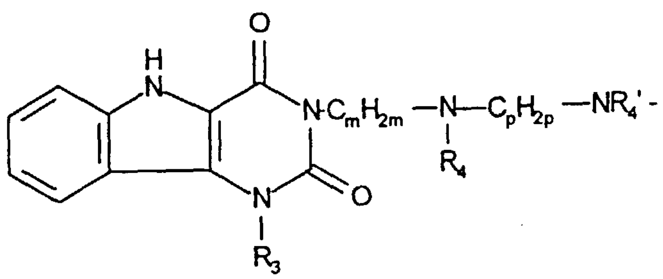

(V')

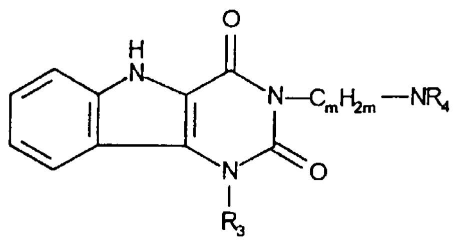

(VI')

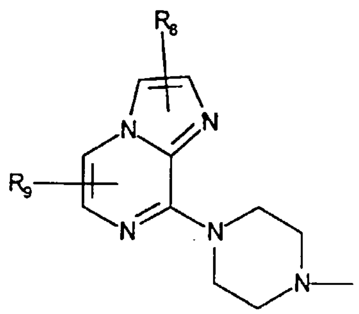

(VII')

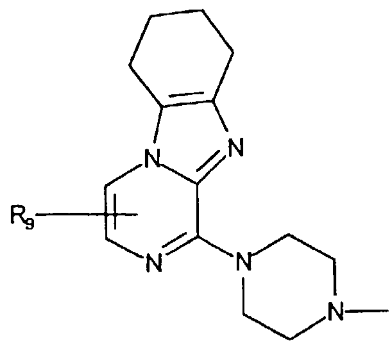

(VIII')

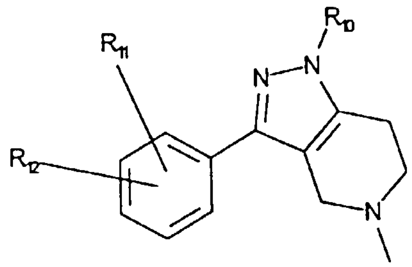

(IX')

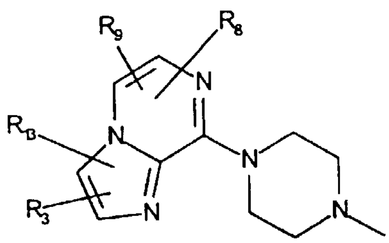

(X')

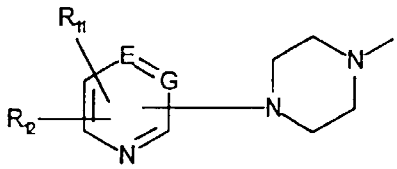

(XI')

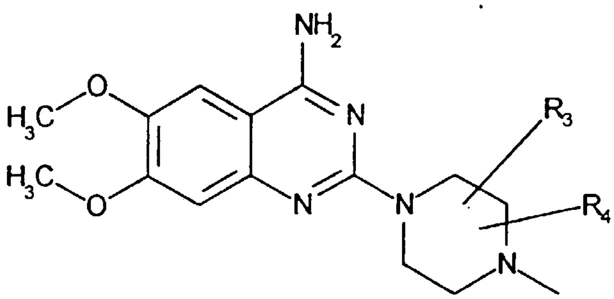

(XII')

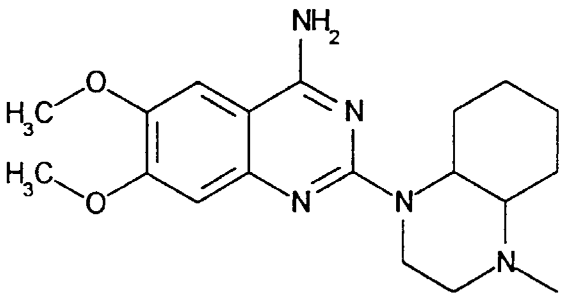

(XIII')

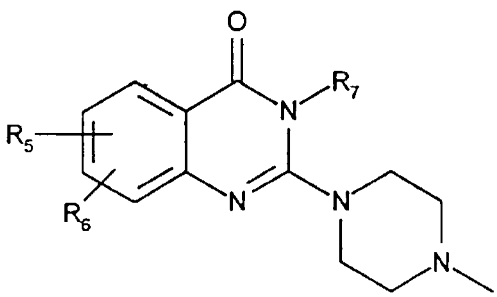

(XIV')

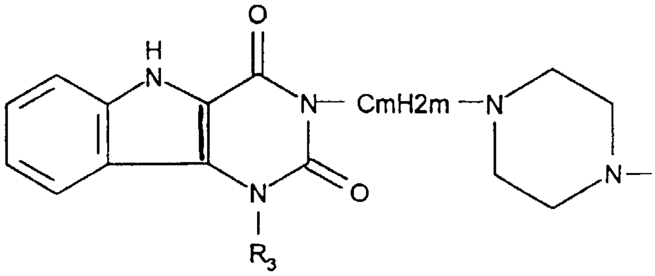

(XV')

**3.** Verbindungen der Formel I, in denen $R_2$ einen Rest der Formel XI bedeutet und G oder E für N stehen.

**4.** Verbindungen der Formel gemäß Anspruch 1

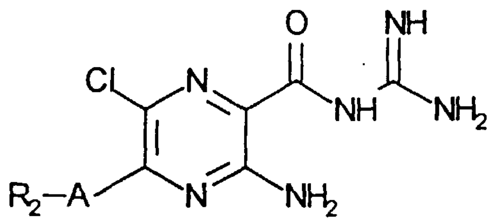

(I a)

worin $R_2$ und A die obige Bedeutung haben.

**5.** Arzneimittel, gekennzeichnet durch einen Gehalt an einer Verbindung nach Anspruch 1 bis 4, neben üblichen Hilfs- und/oder Trägerstoffen.

**6.** Verwendung von Verbindungen nach Anspruch 1 bis 4 bei der Herstellung von Arzneimitteln.

**7.** Verwendung von Verbindungen nach Anspruch 1 bis 4 als Antihypertensiva, Mucolytika, Diuretika, Cancerostatika, zur Behandlung von Krankheiten, die im Zusammenhang mit Ischämien stehen, bei Hirninfarkt, akuten und chronischen Durchblutungsstörungen des Hirns, zur Cardioprotektion und zur Verhinderung von Schäden an Transplantaten.

**8.** Verfahren zur Herstellung der Verbindungen nach Anspruch 1 bis 4 nach konventionellen Methoden, dadurch gekennzeichnet, daß man

(a) eine Verbindung der Formel

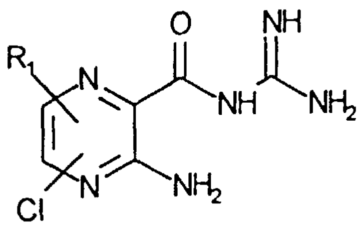

(XVI)

mit einem Amin der Formel

$$R_2 - A - H \qquad (XVII)$$

worin A und $R_2$ die obige Bedeutung haben, umsetzt,
oder daß man

(b) eine Verbindung der Formel

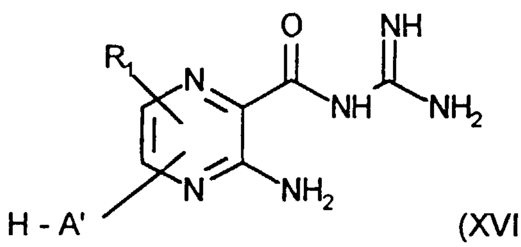

(XVIII)

(wobei A' eine solche Gruppe A ist, die über ein N-Atom an $R_2$ gebunden ist, wie in II', III' und IV') worin $R_1$ die obige Bedeutung hat, mit einer Verbindung der Formel

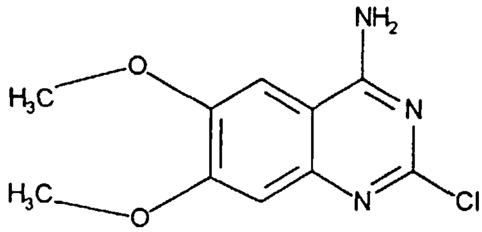

(XIX)

umsetzt, oder daß man

(c) eine Verbindung der Formel

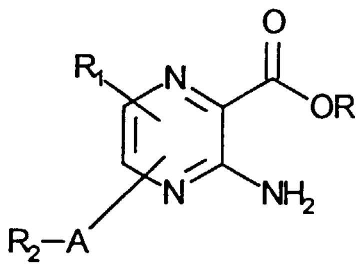

(XX)

worin R einen $C_1$-$C_4$-Alkylrest bedeutet, mit Guanidin umsetzt,

und die erhaltenen Produkte ggf. in sterisch unterschiedliche Formen auftrennt und/oder gewünschtenfalls erhaltene Basen mit Säuren in Salze überführt bzw. erhaltene Salze in freie Basen.

**Claims**

**1.** Compounds of the formula

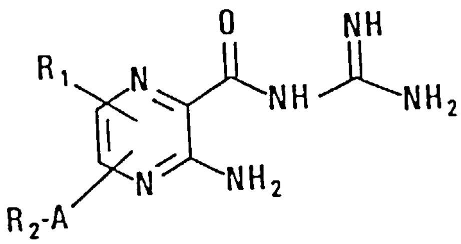

(I)

wherein

A represents one of the following double-bonded groups always bound to the pyrazine carboxamide system via a nitrogen atom:

$-C_mH_{2m}-NR_4-$ , $-C_mH_{2m}-NR_4-C_pH_{2p}-NR_4'-$ , 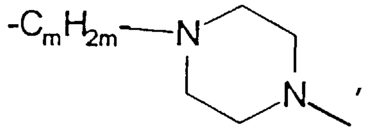 ,

$-NR_4-C_mH_{2m}-NR_4'-$ , 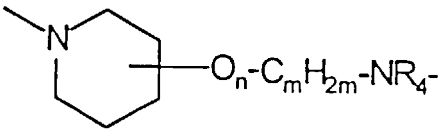 ,

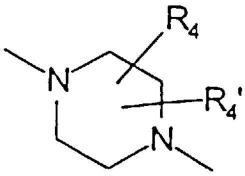 , 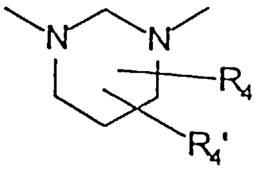 , 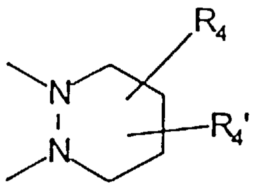 ,

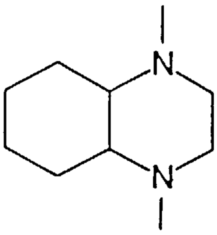 , 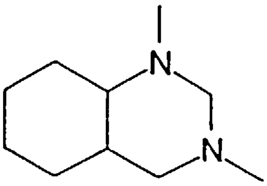 , 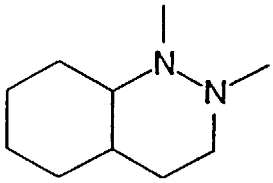 ,

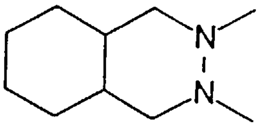 , 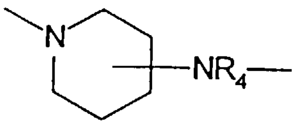 , 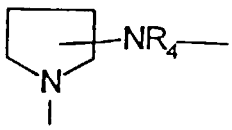 ,

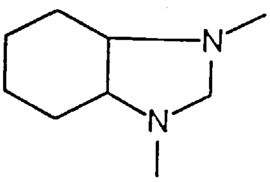 , 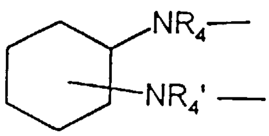 , 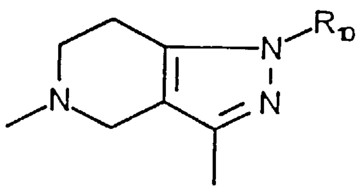

$R_1$ denotes hydrogen, fluorine, chlorine, $C_{1-4}$-alkyl, $C_{1-4}$-alkoxy, trifluoromethyl, phenyl, benzyl, phenyl-lower alkyl, phenyl-lower alkenyl, phenyl-lower alkynyl, or phenoxy; in which any phenyl groups may contain up to three substituents selected from the groups $C_{1-4}$-alkyl, $C_{1-4}$-alkoxy,

$CF_3$, F, Cl or CN or $C_{3-7}$-cycloalkyl;

$R_2$ denotes a group of formula

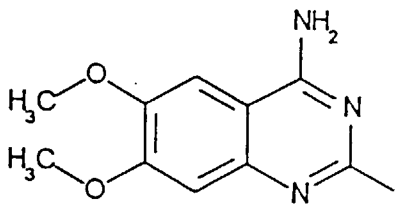

(II)

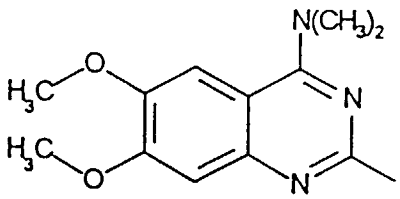

(III)

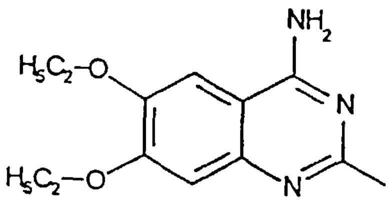

(IV)

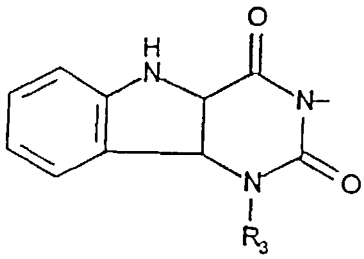

(V)

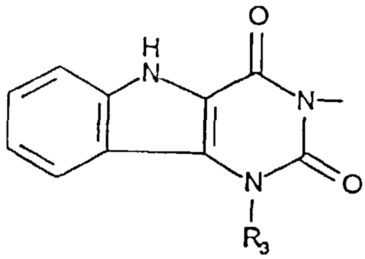

(VI)

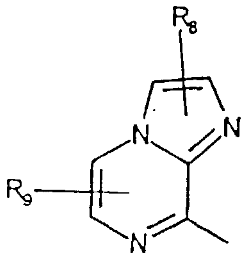

(VII)

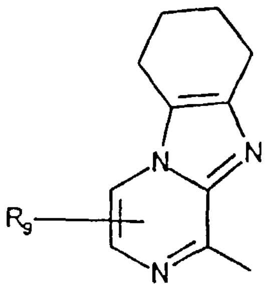

(VIII)

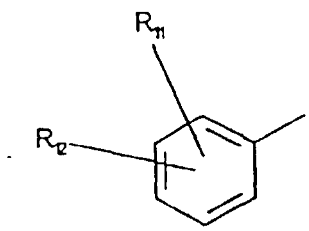

(IX)

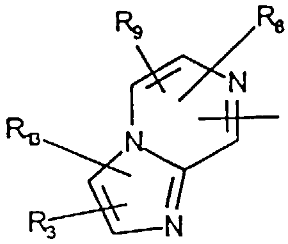

(X)

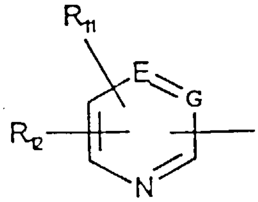

(XI)

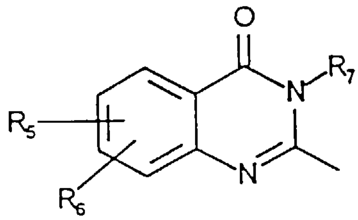

(XII)

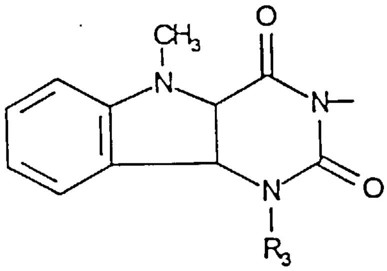

(XIII)

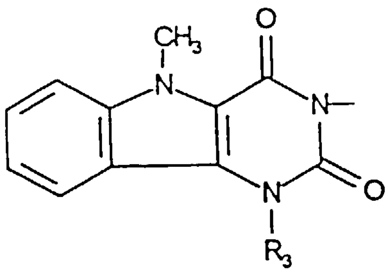

(XIV)

wherein

$R_3$, $R_4$ and $R_4$', which may be the same or different, denote hydrogen or $C_{1-4}$-alkyl, or $R_4$ and $R_4$' may also represent phenyl, benzyl or $C_{3-7}$-cycloalkyl,

$R_5$ and $R_6$, which may be the same or different, denote hydrogen, methyl, methoxy, hydroxy or halogen,

$R_7$ denotes hydrogen, $C_{1-4}$-alkyl, benzyl or benzyloxy,

$R_8$ and $R_9$, which may be the same or different, denote hydrogen, $C_{1-4}$-alkyl, phenyl or halogen,

$R_{10}$ denotes hydrogen or $C_{1-6}$-alkyl, which may also be substituted by phenyl or methyl-, methoxy- or halogen-substituted phenyl,

$R_{11}$ and $R_{12}$, which may be the same or different, denote hydrogen, methyl, methoxy, phenyl, benzyl, nitro, cyano, halogen, trifluoromethyl, amino, $NR_{10}R_{13}$, 1-pyrrolidinyl, 1-pyrazolinyl, 1-imidazolidinyl, 1-piperidinyl, 1-piperazinyl or carbamoyl, and $R_{11}$ may also denote a fused benzene ring which may contain up to three substituents selected from among methyl, methoxy, halogen, $CF_3$ and CN,

$R_{13}$ denotes hydrogen, $C_{1-4}$-alkyl, which may also be substituted by phenyl, phenoxy or benzyloxy, or halogen, and

E and G, which may be the same or different, denote N or CH,

m denotes 2, 3, 4, 5 or 6,

n denotes 0 or 1 and

p denotes 2, 3 or 4,
and the compounds of formula I as bases or as salts and optionally in the form of the individual stereoisomers or cis/trans-isomers or mixtures of such isomers;
with the proviso that when

$R_1$ denotes chlorine and is in the para-position to $NH_2$ substituents of the phenyl system and

A denotes $-C_mH_{2m}-NR_4$ or $HN-CH_2-CH_2-NH$

$R_4$ denotes hydrogen or $C_{1-4}$ alkyl,

$R_2$ may not denote a group of formula (II), (III), (IV), (V), (VI), (VII), (VIII), (X), (XI), (XII), (XIII) or (XIV);
and also with the proviso that when

$R_1$ denotes chlorine and is in the para-position to $NH_2$ substituents of the phenyl system and

A denotes a piperazine ring

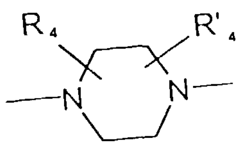

in which $R_4$ and $R_4$' denote hydrogen,

$R_2$ may not denote a group of formula (II), (III), (IV), (V), (VI), (VII), (VIII), (X), (XI), (XII), (XIII) or (XIV);
and also with the proviso that when

$R_1$ denotes chlorine and is in the para-position to $NH_2$ substituents of the phenyl system,

A denotes a $-CH_2-CH_2-NH$ bridge

$R_2$ may not denote phenyl.

**2.** Compounds of formula (I), wherein $R_2$-A denotes a group of formulae II' to XV' and $R_3$, $R_4$ and $R_4$', which may be the same or different, denote hydrogen or a $C_{1-3}$-alkyl group

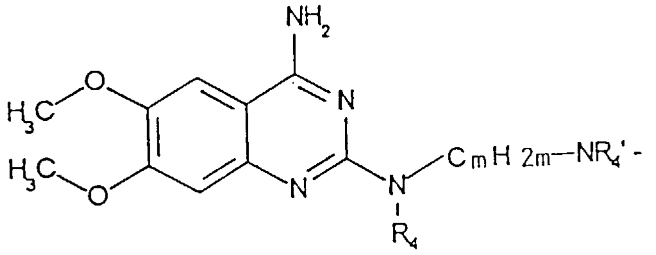

(II')

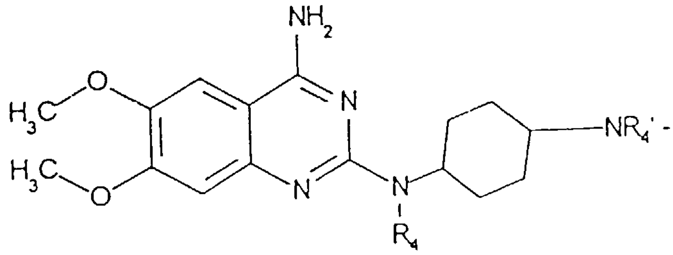

(III')

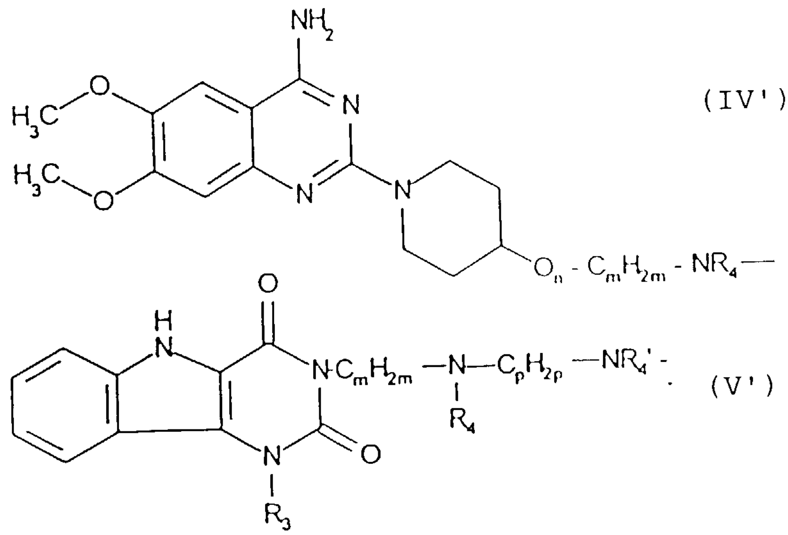

(IV')

(V')

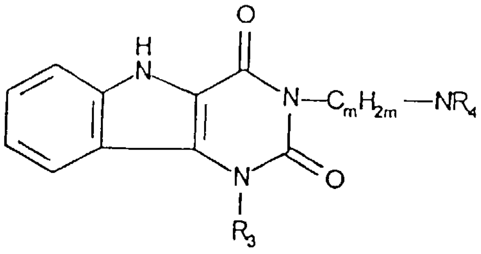

(VI')

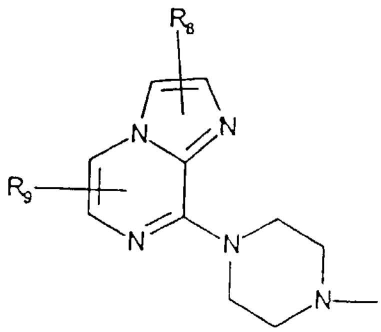

(VII')

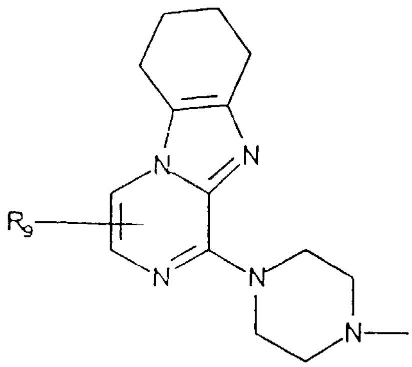

(VIII')

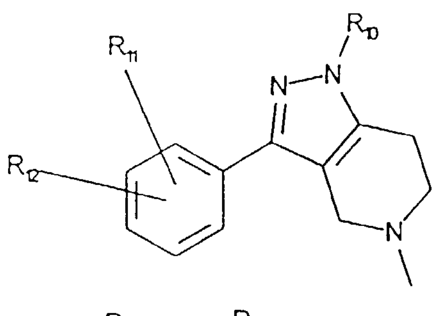

(IX')

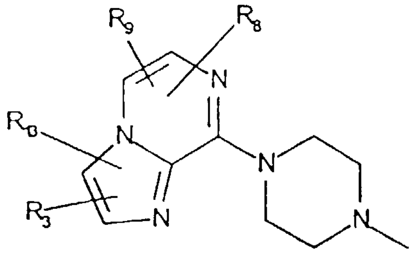

(X')

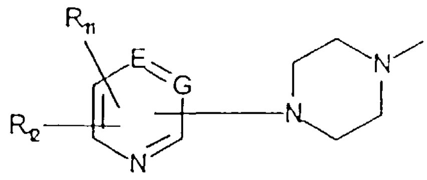

(XI')

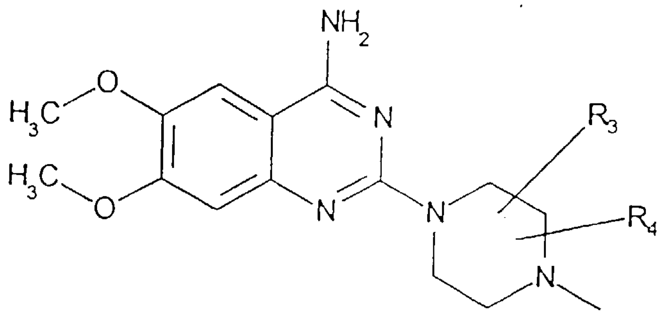

(XII')

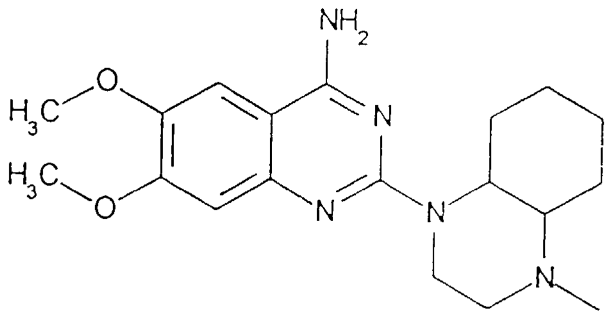

(XIII')

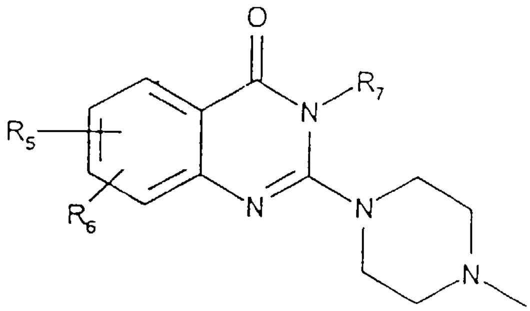

(XIV')

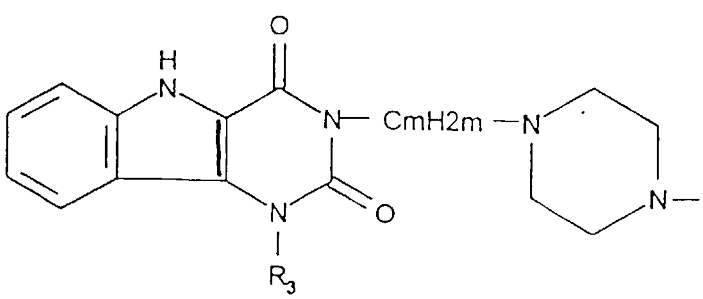

(XV')

**3.** Compounds of formula (I), wherein $R_2$ is a group of formula XI and G or E represent N.

**4.** Compounds of formula

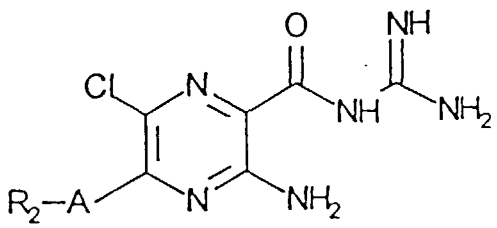

(Ia)

according to claim 1, wherein $R_2$ and A are as hereinbefore defined.

**5.** Pharmaceutical compositions characterised in that they contain a compound as claimed in any of claims 1 to 4 in admixture with conventional excipients and/or substrates.

**6.** Use of compounds according to claims 1 to 4 in the preparation of pharmaceutical compositions.

**7.** Use of a compound as claimed in any of claims 1 to 4 as an antihypertensive, a mucolytic, a diuretic, a cancerostatic, for treating diseases connected with ischaemia, in cerebral infarction, for acute and chronic circulatory disorders of the brain, for cardioprotection and for preventing damage to transplants.

**8.** A process for preparing a compounds according to claims 1 to 4 by conventional methods, characterised in that

(a) a compound of formula

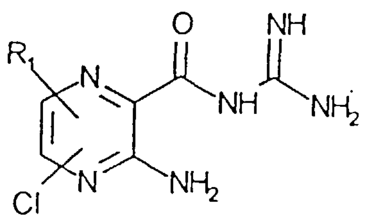

(XVI)

is reacted with an amine of formula

$$R_2 - A - H \qquad (XVII)$$

wherein A and $R_2$ are as hereinbefore defined,
or

(b) a compound of formula

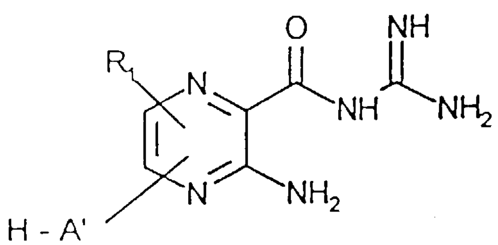

(XVIII)

(wherein A' is a group A which is bound via an N-atom to $R_2$, as in II', III' and IV')
wherein $R_1$ is as hereinbefore defined,
is reacted with a compound of formula

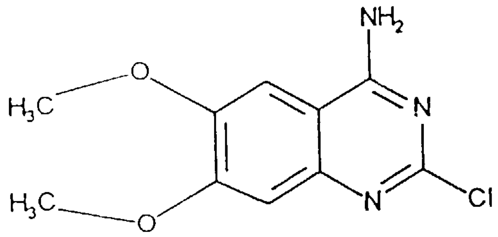

(XIX)

or

(c) a compound of formula

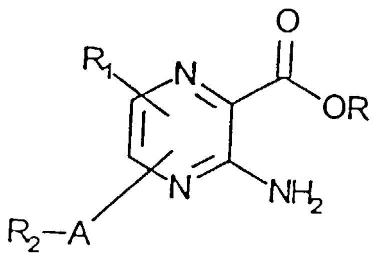

(XX)

wherein R is a $C_{1-4}$ alkyl group, and is reacted with guanidine,

and the products obtained are, if required, separated into sterically different forms and/or any bases obtained are converted into salts or any salts obtained are converted into free bases as desired.

**Revendications**

**1.** Composés de formule

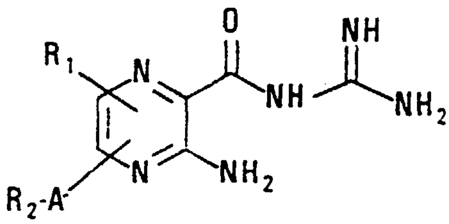

(I)

où

A représente l'un des groupes divalents liés toujours par un atome d'azote au système pyrazinecarboxamide

$-C_mH_{2m}-NR_4-$ $-C_mH_{2m}-NR_4-C_pH_{2p}-NR_4'-$ $-NR_4-C_mH_{2m}-NR_4'$

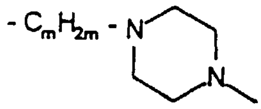

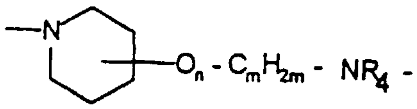

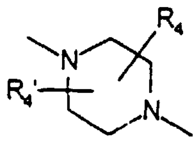

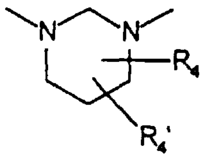

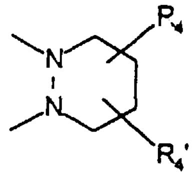

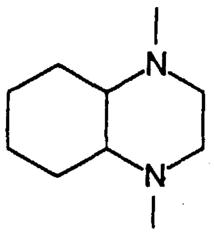
,
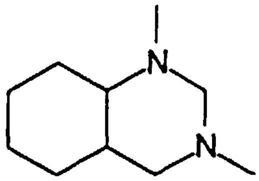
,
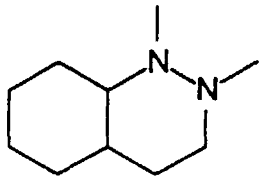
,

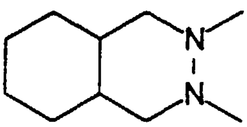
,
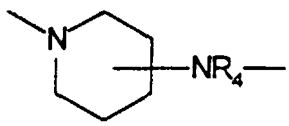
,
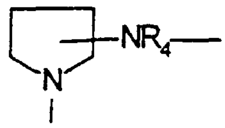
,

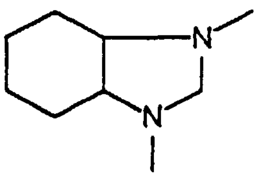
,
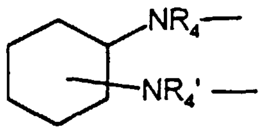
,
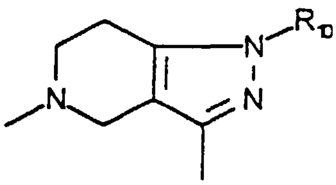

$R_1$ représente hydrogène, fluor, chlore, alkyle en $C_1$-$C_4$, alcoxy en $C_1$-$C_4$, trifluorométhyle, phényle, benzyle, phényl-alkyle intérieur, phényl-alcényle inférieur, phényl-alcynyle inférieur, phénoxy, où les différents groupes phényle peuvent présenter jusqu'à trois substituants du groupe de alkyle en $C_1$-$C_4$, alcoxy en $C_1$-$C_4$, $CF_3$, F, Cl, CN, ou cycloalkyle en $C_3$-$C_7$;

$R_2$ représente un reste de formule

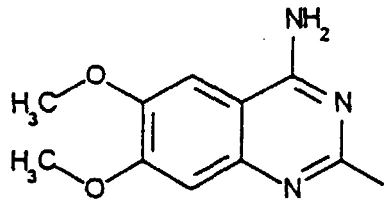

(II)

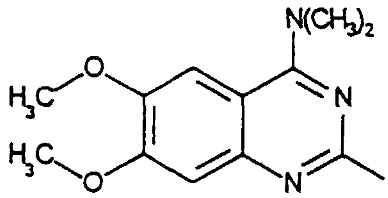

(III)

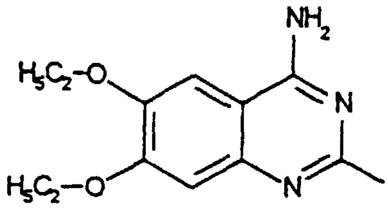

(IV)

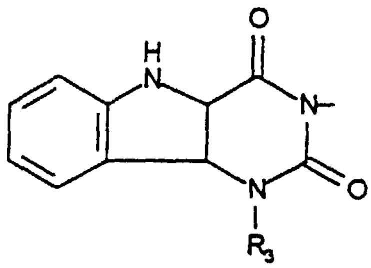

(V)

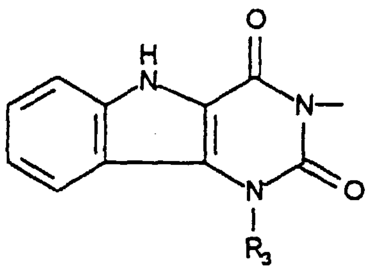

(VI)

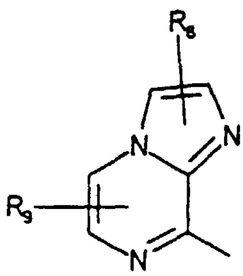

(VII)

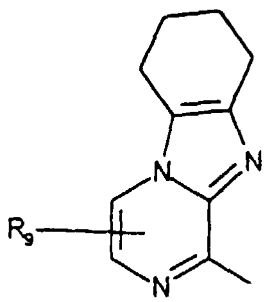

(VIII)

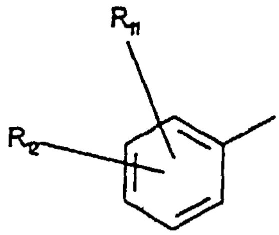

(IX)

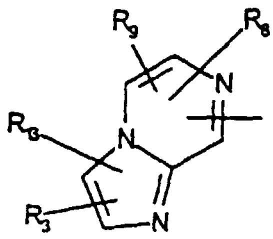

(X)

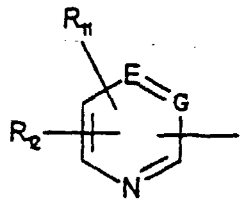

(XI)

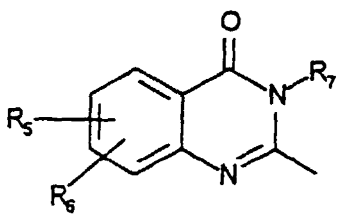

(XII)

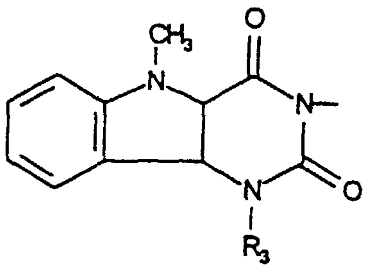

(XIII)

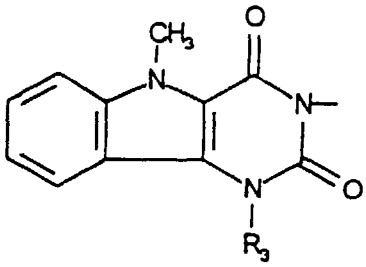

(XIV)

où

$R_3$, $R_4$ et $R_4$', qui peuvent être identiques ou différents, représentent hydrogène, alkyle en $C_1$-$C_4$, $R_4$ et $R_4$' représentent aussi phényle, benzyle et cycloalkyle en $C_3$-$C_7$,

$R_5$ et $R_6$, qui peuvent être identiques ou différents, représentent hydrogène, méthyle, méthoxy, hydroxyle ou halogène,

$R_7$ représente hydrogène, alkyle en $C_1$-$C_4$, benzyle ou benzyloxy,

$R_8$ et $R_9$, qui peuvent être identiques ou différents, représentent hydrogène, alkyle en $C_1$-$C_4$, phényle ou halogène,

$R_{10}$ représente hydrogène ou alkyle en $C_1$-$C_6$, qui peut aussi être substitué par phényle ou méthyle, méthoxy ou phényle halogénosubstitué,

$R_{11}$ et $R_{12}$, qui peuvent être identiques ou différents, représentent hydrogène, méthyle, méthoxy, phényle, benzyle, nitro, cyano, halogène, trifluorométhyle, amino, $NR_{10}R_{13}$, 1-pyrrolidinyle, 1-pyrazolinyle, 1-imidazolidinyle, 1-pipéridinyle, 1-pipérazinyle ou carbamoyle, $R_{11}$ peut aussi comporter un cycle benzénique condensé qui peut présenter jusqu'à trois substituants du groupe de méthyle, méthoxy, halogène, $CF_3$ et CN,

$R_{13}$ représente hydrogène, alkyle en $C_1$-$C_4$ qui peut aussi être substitué par phényle, phénoxy ou benzyloxy, ou halogène, et

E et G, qui peuvent être identiques ou différents, représentent N ou CH,

m représente 2, 3, 4, 5 ou 6,

n représente 0 ou 1 et

p représente 2, 3 ou 4

et les composés de formule I peuvent être sous forme de bases ou de sels et éventuellement sous forme des différents stéréoisomères ou isomères cis/trans ou des mélanges de tels isomères

à condition que si

$R_1$ représente le chlore et se trouve en position para du substituant $NH_2$ sur le système phényle et

A a la signification de $-C_mH_{2m}-NR_4-$ ou $HN-CH_2-CH_2-NH$

$R_4$ représente hydrogène ou alkyle en $C_1$-$C_4$ (IV), (V), (VI), (VII), (VIII), (X), (XI), (XII), (XIII) ou

$R_2$ ne doit pas représenter un reste de formule (II), (III), (XIV);

ainsi qu'à condition que si

$R_1$ représente le chlore et se trouve en position para du substituant $NH_2$ sur le système phényle et

A représente un cycle pipérazine

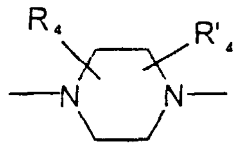

où $R_4$ et $R_4$' représentent l'hydrogène

$R_2$ ne doit pas représenter un reste de formule (II), (III), (IV), (V), (VI), (VII), (VIII), (X), (XI), (XII), (XIII) ou (XIV); ainsi qu'à condition que si

$R_1$ représente le chlore et se trouve en position para du substituant $NH_2$ sur le système phényle

A représente un pont $-CH_2-CH_2-NH-$

$R_2$ ne doit pas avoir la signification de phényle.

**2.** Composés de formule I où $R_2$-A représente un reste des formules II' à XV', $R_3$, $R_4$ et R4', qui peuvent être identiques ou différents, représentent l'hydrogène ou un reste alkyle en $C_1$-$C_3$

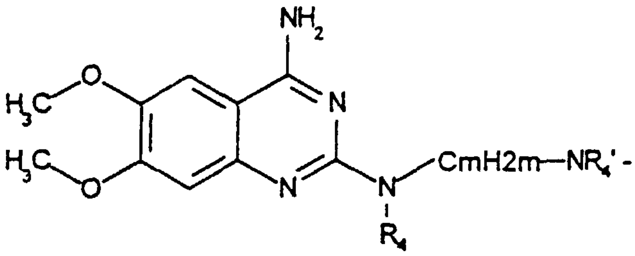

(II')

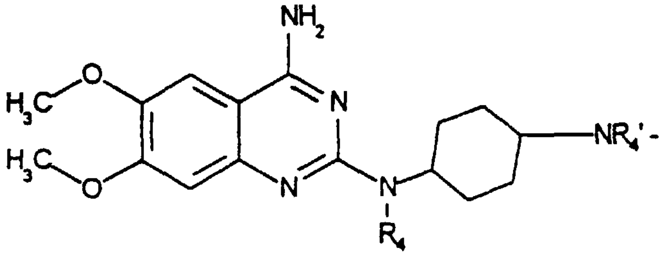

(III')

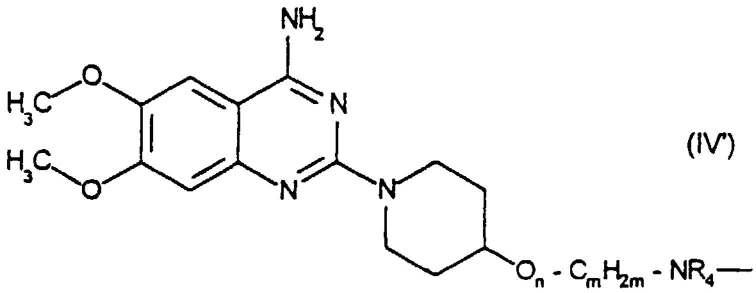

(IV')

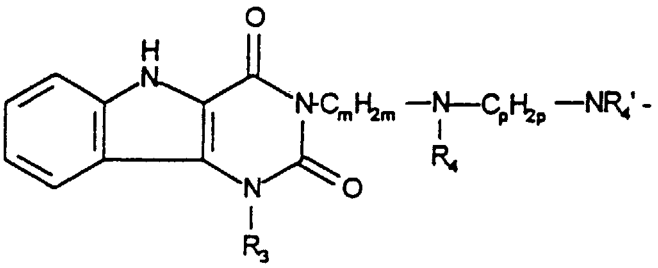

(V')

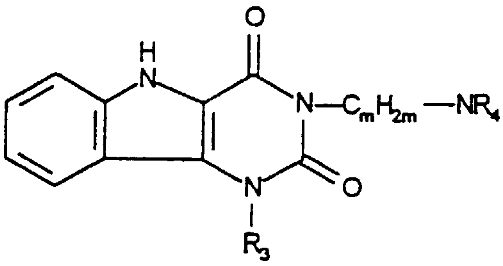

(VI')

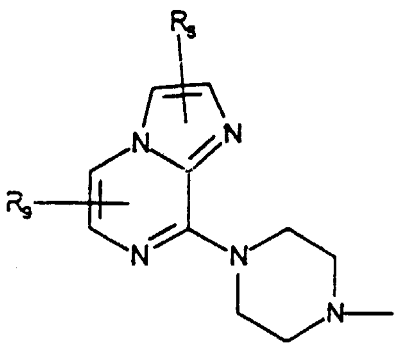

(VII')

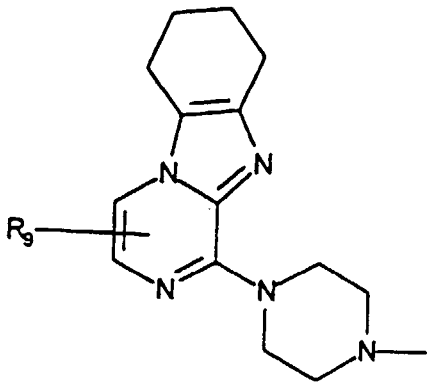

(VIII')

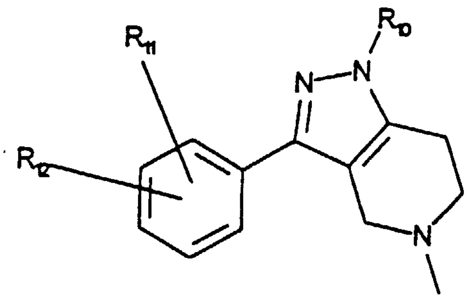

(IX')

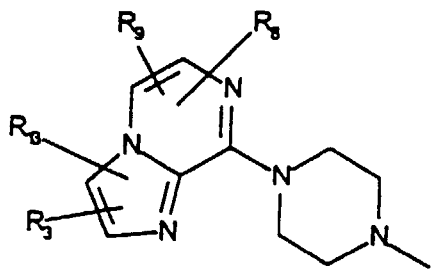

(X')

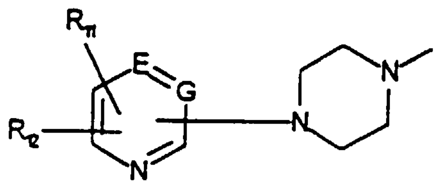

(XI')

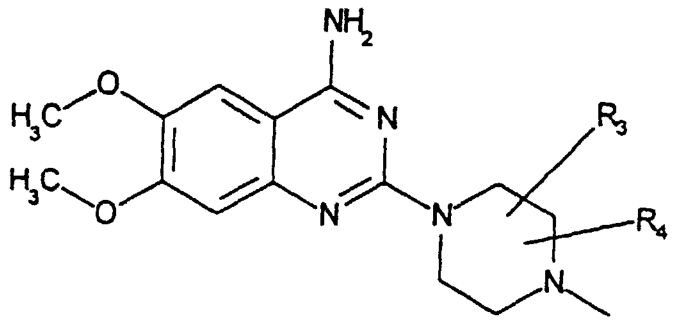

(XII')

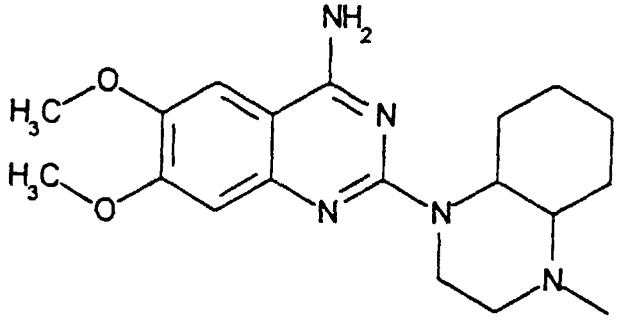

(XIII')

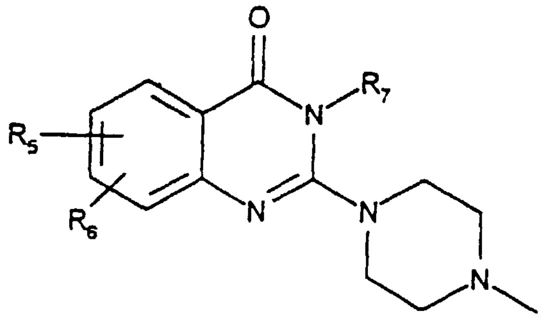

(XIV')

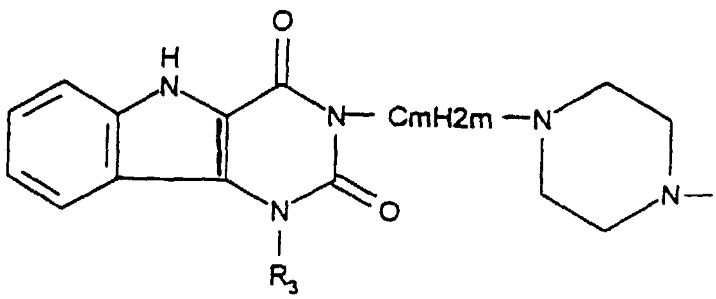

(XV')

**3.** Composés de formule I où $R_2$ représente un reste de formule XI et G ou E représentent N.

**4.** Composés de formule selon la revendication 1

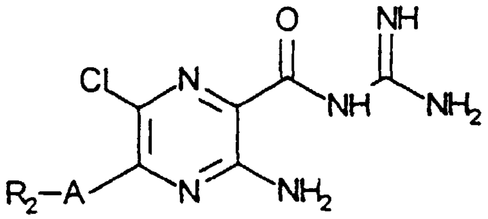

(I a)

où $R_2$ et A ont la signification ci-dessus.

**5.** Médicament caractérisé par une teneur en un composé selon les revendications 1 à 4 outre des adjuvants et/ou supports courants.

**6.** Utilisation de composés selon les revendications 1 à 4 lors de la production de médicaments.

**7.** Utilisation de composés selon les revendications 1 à 4 comme antihypertenseurs, mucolytiques, diurétiques, cancérostatiques, pour le traitement des maladies qui sont liées à des ischémies, dans l'infarctus cérébral, les troubles aigus et chroniques de l'irrigation sanguine du cerveau, pour la cardioprotection et pour éviter des lésions sur les greffes.

**8.** Procédé de préparation des composés selon les revendications 1 à 4 d'après des méthodes conventionnelles, caractérisé en ce que

(a) on fait réagir un composé de formule

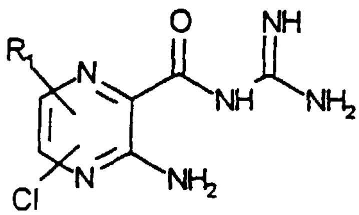

(XVI)

avec une amine de formule

$$R_2 - A - H \qquad (XVII)$$

où A et $R_2$ ont la signification indiquée ci-dessus,
ou bien en ce que
(b) on fait réagir un composé de formule

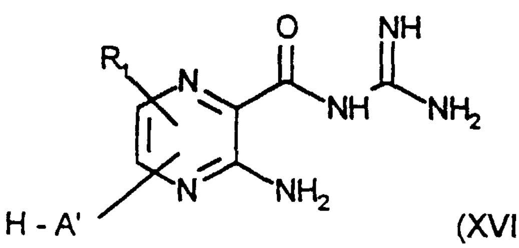

(XVIII)

(où A' est un groupe A qui est lié à $R_2$ par un atome d'azote, comme dans II', III' ou IV')
où $R_1$ a la signification ci-dessus,

avec un composé de formule

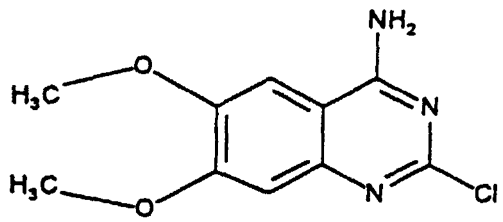

(XIX)

ou bien en ce que

(c) on fait réagir un composé de formule

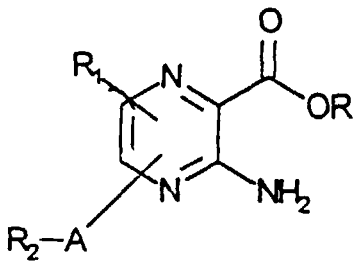

(XX)

où R représente un reste alkyle en $C_1$-$C_4$,
avec la guanidine,

et on résout éventuellement les produits obtenus en des formes stériquement différentes et/ou si on le souhaite on convertit les bases obtenues en sels avec des acides ou les sels obtenus en bases libres.